(19)

**Europäisches Patentamt**
**European Patent Office**
**Office européen des brevets**

(11) **EP 4 620 494 A1**

(12) **EUROPEAN PATENT APPLICATION**
published in accordance with Art. 153(4) EPC

(43) Date of publication:
**24.09.2025 Bulletin 2025/39**

(21) Application number: **23892024.3**

(22) Date of filing: **15.11.2023**

(51) International Patent Classification (IPC):
*A61L 27/38* (2006.01)     *A61L 27/36* (2006.01)

(52) Cooperative Patent Classification (CPC):
**A61L 27/36; A61L 27/38**

(86) International application number:
**PCT/KR2023/018404**

(87) International publication number:
**WO 2024/106959 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:   **15.11.2022   KR 20220153034**

(71) Applicants:
• **Seoul National University Hospital**
  **Seoul 03080 (KR)**
• **Seoul National University R&DB Foundation**
  **Seoul 08826 (KR)**

(72) Inventors:
• **KWON, Seong Keun**
  **Seoul 06004 (KR)**
• **CHOI, Ji Suk**
  **Seoul 02583 (KR)**
• **SON, Young Ju**
  **Seoul 03080 (KR)**
• **EOM, Min Rye**
  **Seoul 01173 (KR)**
• **JEONG, Eun Ji**
  **Seoul 08026 (KR)**

(74) Representative: **Nederlandsch Octrooibureau**
**P.O. Box 29720**
**2502 LS The Hague (NL)**

(54) **COMPOSITION FOR PROMOTING TISSUE REGENERATION COMPRISING STEM CELL SPHEROIDS**

(57)   The present invention relates to a composition for promoting tissue regeneration, the composition comprising stem cell spheroids. The stem cell spheroids according to the present invention can be produced in a uniform size and are standardized, the speed of tissue regeneration is faster than that of 2D monolayer cultured stem cells, and the regenerated tissue showed higher similarity with the native tissue. The stem cell spheroids according to the present invention have the excellent effect on the regeneration of various tissues such as salivary glands, trachea, vocal cords, muscles, skin, and fat through inducing angiogenesis, and thus are expected to be useful as a cell therapy, for various tissue diseases, and the like.

**FIG. 7B**

scale bar: 20μm

EP 4 620 494 A1

## Description

[Technical Field]

The present invention relates to a composition for promoting tissue regeneration comprising stem cell spheroids.

This application claims priority to and the benefit of Korean Patent Application No. 10-2022-0153034, filed on November 15, 2022, and Korean Patent Application No. 10-2023-0158492, filed on November 15, 2023, the disclosure of which is incorporated herein by reference in its entirety.

[Background Art]

Recently, with the growing interest in stem cell-related technology, it is recognized that pluripotent stem cells, which have the ability to form various organs through proliferation and differentiation, can fundamentally solve most diseases as well as organ damage.

Stem cells are cells that can differentiate into various cells constituting biological tissue, and are a general term for undifferentiated cells that can be obtained from each tissue of an embryo, fetus or adult before differentiation. Stem cells are characterized by the ability to differentiate into specific cells in response to differentiation cues (environment) to produce cells identical to themselves through cell division (self-renewal) and to exhibit the plasticity to differentiate into different cells according to differentiation cues. Stem cells may be classified into pluripotent, multipotent, totipotent, and unipotent stem cells according to their differentiation ability. Pluripotent stem cells are cells that have the potential to differentiate into all cells, and some stem cells have the potential to be multipotent or unipotent. Stem cells have the ability to promote the growth and differentiation of surrounding cells through the expression of various bioactive factors (paracrine effect), and are receiving much attention in the field of regenerative medicine, which is closely related to intractable and degenerative diseases or organ regeneration, as a raw material for cell therapy.

Much research and development has been actively conducted in relation to the application of these stem cells to cell therapy using their differentiation ability, and through various studies, many possibilities for restoring cell and tissue functions through their application to cell therapy in various diseases requiring the restoration and regeneration of lost cells, such as nerve diseases, heart diseases, lung diseases, liver diseases, and carcinectomy, are being proposed.

Generally, a cell therapy product is a drug that is produced by manipulating cells using a physical, chemical, or biological method, for example, in vitro culture or screening, and most cell therapy products are developed in the form of a suspension of single cells grown in vitro in an additive. However, the single cells in the suspension have limitations in that transplanted cells cannot directly form regenerative tissue due to the physical and chemical stress of the transplantation environment and cell-substrate and cell-cell detachment-induced apoptosis, called anoikis, and only indirect effects caused by materials secreted by the cells can be expected.

To overcome these limited therapeutic effects, the development of a spheroid-type cell therapy product is being actively developed to increase cell viability after in vivo transplantation and to raise the therapeutic effects of cell therapy, such as an antiinflammatory effect and a vascular regenerative effect, by re-aggregating and recombining cells using a three-dimensional culture method. The technology for preparing cell spheroids is a common technology that is widely used, especially, in the field of anticancer drug development, and is also used in the field of tissue regeneration/reconstruction due to three-dimensional cell culture. However, there is a lack of the research on the standardization of cell spheroids with the most optimal condition, which express the bioactive factors necessary for tissue regeneration/reconstruction, and the confirmation of regenerative effects on various tissues using them.

Therefore, the present inventors attempted to fabricate standardized stem cell spheroids of uniform size and utilize them to develop cell therapy products that can promote the regeneration of various tissues.

[Disclosure]

[Technical Problem]

The present invention has been devised to solve the above-described problems of the conventional technologies, and an object of the present invention is to provide a composition or a cell therapeutic agent for promoting tissue regeneration comprising a stem cell spheroid as an active ingredient.

However, technical problems to be solved in the present invention are not limited to the above-described problems, and other problems which are not described herein will be fully understood by those of ordinary skill in the art from the following descriptions.

[Technical Solution]

To achieve the above object, the present invention provides a composition for promoting tissue regeneration, comprising stem cell spheroids as an active ingredient.

In one embodiment of the present invention, the stem cells may be one or more selected from the group consisting of bone marrow-derived mesenchymal stem cells, adipose-derived mesenchymal stem cells, embryonic stem cell-derived mesenchymal stem cells, tonsil-derived mesenchymal stem cells, and induced pluripotent stem cell-derived mesenchymal stem cells, but are not limited thereto.

In another embodiment of the present invention, the tissue may be one or more selected from the group consisting of skin, fat, salivary glands, the trachea, airways, the vocal folds, and muscles, but is not limited thereto.

In yet another embodiment of the present invention, the composition may further comprise a scaffold for tissue engineering, but is not limited thereto.

In yet another embodiment of the present invention, the stem cell spheroids may exhibit enhanced expression of bioactive factors associated with the promotion of tissue regeneration, but is not limited thereto.

In yet another embodiment of the present invention, the bioactive factors may be one or more selected from the group consisting of vascular endothelial growth factors (VEGFs), matrix metalloproteinases (MMPs), fibroblast growth factors (FGFs), epidermal growth factors (EGFs), hepatocyte growth factor (HGF), platelet-derived growth factor (PDGF), placental growth factor (PlGF), granulocyte-macrophage colony-stimulating factor (GM-CSF), transforming growth factor beta (TGF-$\beta$), and angiopoietin, but are not limited thereto.

In yet another embodiment of the present invention, the stem cells may promote angiogenesis, but are not limited thereto.

In yet another embodiment of the present invention, the stem cell spheroids may have diameters ranging from 100 to 500 $\mu$m, but are not limited thereto.

Additionally, the present invention provides a cell therapeutic agent for promoting tissue regeneration, comprising stem cell spheroids as an active ingredient.

Additionally, the present invention provides a method for promoting tissue regeneration, comprising administering a composition comprising stem cell spheroids as an active ingredient to a subject in need thereof.

Additionally, the present invention provides a use of a composition comprising stem cell spheroids as an active ingredient for the promotion of tissue regeneration.

Additionally, the present invention provides a use of a composition comprising stem cell spheroids as an active ingredient for the preparation of a formulation for promoting tissue regeneration.

[Advantageous Effects]

The stem cell spheroid according to the present invention can be manufactured with a uniform size and is standardized, exhibits faster tissue regeneration compared to two-dimensionally cultured stem cells, and the regenerated tissue has high similarity to the original tissue. The stem cell spheroid according to the present invention exhibits excellent effects on the regeneration of various tissues such as salivary gland, trachea, vocal cord, muscle, skin, and fat tissue through actions such as angiogenesis, and is expected to be usefully utilized as a cell therapeutic agent for various tissue disorders.

[Description of Drawings]

FIG. 1 shows the result of confirming the diameter of the stem cell spheroid according to the number of stem cells in one embodiment of the present invention.

FIG. 2 shows the result of analyzing the correlation among the diameter of a single cell, the diameter of the stem cell spheroid, and the initial number of single cells by logistic regression analysis in one embodiment of the present invention. The blue dotted line in FIG. 2 represents the regression line.

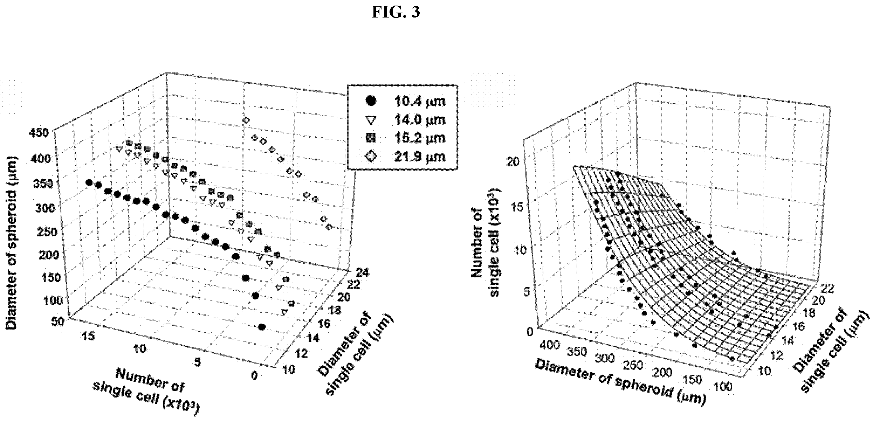

FIG. 3 shows the result of representing the correlation among the diameter of a single cell, the diameter of the stem cell spheroid, and the initial number of single cells as a 3D graph in one embodiment of the present invention. The left diagram of FIG. 3 shows the diameter of the stem cell spheroid according to the diameter and number of single cells, where the black dots represent single cells with a diameter of 10.4 $\mu$m, the white dots represent single cells with a diameter of 14.0 $\mu$m, the red dots represent single cells with a diameter of 15.2 $\mu$m, the green dots represent single cells with a diameter of 21.9 $\mu$m, and the yellow dots represent single cells with a diameter of 23.7 $\mu$m. The right diagram of FIG. 3 shows the diameter of a single cell (x) and the diameter of the spheroid (y) for predicting the required number of single cells (f) for manufacturing the stem cell spheroid, and the blue mesh indicates the regression (3D, Gaussian).

FIG. 4 shows the result of applying the function for calculating the required number of single cells in various culture vessels in one embodiment of the present invention.

FIG. 5A shows the result of observing the degree of hypoxia inside the spheroid according to the diameter of the stem cell spheroid by fluorescence microscopy in one embodiment of the present invention.

FIG. 5B shows the result of confirming the cell viability according to the diameter of the stem cell spheroid in one embodiment of the present invention.

FIG. 5C shows the result of confirming the cumulative secretion amount of bioactive factors according to the diameter of the stem cell spheroid by ELISA in one embodiment of the present invention.

FIG. 5D shows the result of confirming the expression of various bioactive factors related to angiogenesis by treatment with the stem cell spheroid in one embodiment of the present invention (blue line: a reference line standardized to 1 based on the amount of each bioactive factor secreted by two-dimensionally cultured stem cells).

FIG. 6A shows the result of confirming the tube formation-inducing efficacy of the stem cell spheroid through co-culture of the stem cell spheroid and GFP-HUVEC in one embodiment of the present invention.

FIG. 6B shows a diagram quantitatively representing the result of FIG. 6A.

FIG. 7A shows the result of H&E staining of salivary gland tissue after injection of the stem cell spheroid following radiation irradiation in one embodiment of the present invention.

FIG. 7B shows the result of confirming the mucin secretion function of the salivary gland after injection of the stem cell spheroid following radiation irradiation in one embodiment of the present invention.

FIG. 7C shows the result of functional and morphological evaluation of the salivary gland after injection of the stem cell spheroid following radiation irradiation in one embodiment of the present invention.

FIG. 8A shows the attachment of two-dimensionally cultured stem cells and stem cell spheroids applied to the inside and outside of the tracheal scaffold in one embodiment of the present invention.

FIG. 8B shows the implantation of a tracheal scaffold applied with two-dimensionally cultured stem cells and stem cell spheroids into a tracheal partial defect animal model and the appearance of the implanted tracheal scaffold 14 weeks after implantation in one embodiment of the present invention.

FIG. 8C shows an endoscopic image of the airway after implantation of a tracheal scaffold applied with two-dimensionally cultured stem cells and stem cell spheroids in one embodiment of the present invention.

FIG. 8D shows the result of evaluating the efficacy of mucosal structural reconstruction in the airway after implantation of a tracheal scaffold applied with two-dimensionally cultured stem cells and stem cell spheroids in one embodiment of the present invention.

FIG. 8E shows the result of evaluating the efficacy of mucosal functional reconstruction in the airway after implantation of a tracheal scaffold applied with two-dimensionally cultured stem cells and stem cell spheroids in one embodiment of the present invention.

FIG. 8F shows the result of evaluating the regenerated airway mucosal tissue inside the scaffold (left panel of FIG. 8F) and the angiogenesis efficacy in the muscle layer covering the trachea outside the scaffold (right panel of FIG. 8F) after implantation of a tracheal scaffold applied with two-dimensionally cultured stem cells and stem cell spheroids in one embodiment of the present invention.

FIG. 9 shows the result of confirming the vocal fold reconstruction effect of the stem cell spheroid on aged vocal folds in one embodiment of the present invention.

FIG. 10A shows the application of Matrigel containing the stem cell spheroid over the damaged vessel after generating an animal model of ischemic hindlimb disease in one embodiment of the present invention.

FIG. 10B shows the result of measuring blood flow velocity after induction of tissue regeneration by transplantation of the stem cell spheroid in an animal model of ischemic hindlimb disease in one embodiment of the present invention.

FIG. 10C shows the blood flow and the appearance of the foot after induction of tissue regeneration by transplantation of the stem cell spheroid in an animal model of ischemic hindlimb disease in one embodiment of the present invention.

FIG. 10D shows the result of H&E staining of a cross-section of the thigh muscle after induction of tissue regeneration by transplantation of the stem cell spheroid in an animal model of ischemic hindlimb disease in one embodiment of the present invention.

FIG. 10E shows the result of confirming the delayed damage and cross-sectional area (CSA) of regenerated muscle fibers after transplantation of the stem cell spheroid in an animal model of ischemic hindlimb disease in one embodiment of the present invention.

FIG. 10F shows the result of confirming the vascular distribution in the regenerated muscle after transplantation of the stem cell spheroid in an animal model of ischemic hindlimb disease in one embodiment of the present invention (BF: Bicep Femoris, CF: Caudofemoralis, SM: Semimembranosus).

FIG. 11A shows the appearance after treatment of a skin defect site with a stem cell spheroid in one embodiment of the present invention.

FIG. 11B shows the result of H&E staining of tissue after treatment of a skin defect site with a stem cell spheroid in one embodiment of the present invention.

FIG. 12 shows the result of H&E staining and Oil Red O staining of regenerated adipose tissue after transplantation of the stem cell spheroid into the fat pad of an animal in one embodiment of the present invention.

[Modes of the Invention]

The inventors confirmed the regenerative effects on various tissues such as salivary gland, trachea, vocal cord, muscle, skin, and fat tissue by manufacturing stem cell spheroids that can be produced with a uniform size and are standardized. It was confirmed that the stem cell spheroid has the effect of regenerating or reconstructing the above tissues through actions such as angiogenesis. It was also confirmed that the tissue regeneration is faster and the regenerated tissue has high similarity to the original tissue compared to two-dimensionally cultured stem cells. Additionally, it was confirmed that when the stem cell spheroid is used in combination with a scaffold for tissue engineering, the expression of bioactive factors promoting tissue regeneration is enhanced, thereby improving the efficacy of tissue regeneration.

Hereinafter, the present invention will be described in detail.

The present invention provides a composition for promoting tissue regeneration, comprising stem cell spheroids as an active ingredient.

In the present invention, a "stem cell" refers to a cell that forms the basis of cells or tissues constituting an organism, and is characterized by the ability to self-renew through repeated division and the multipotency to differentiate into specific functional cells depending on the environment. Stem cells arise from all tissues during fetal development and are also found in certain adult tissues such as bone marrow and epithelial tissues where active cell replacement occurs. Stem cells are classified according to their differentiation potential into totipotent stem cells, which are formed when the fertilized egg begins its first division, pluripotent stem cells located in the inner cell mass of the blastocyst that results from continued division, and multipotent stem cells that exist within mature tissues and organs. Multipotent stem cells refer to cells that can differentiate only into specific cells of the tissue or organ in which they reside, and they are involved not only in the growth and development of each tissue and organ during the fetal, neonatal, and adult stages, but also in maintaining homeostasis of adult tissues and inducing regeneration in the event of tissue damage. Such tissue-specific multipotent cells are collectively referred to as adult stem cells.

Mesenchymal stem cells, classified as adult stem cells, are well known as repair cells of various connective tissues and are capable of differentiating into various types of mesenchymal lineage cells. Additionally, due to the advantages of easy acquisition and clinical applicability, they are attracting attention as a material for developing therapeutics for neurological diseases and for use in regenerative medicine, as they can compensate for the disadvantages of neural stem cells, which are located deep in the brain, are difficult to obtain in sufficient quantities, and carry the risk of brain damage. They can be harvested from tissues such as bone marrow, umbilical cord blood, adipose tissue, and umbilical cord, and unlike hematopoietic stem cells, they possess the ability to differentiate into various human tissue-forming cells such as adipocytes, osteocytes, chondrocytes, neurons, and cardiomyocytes.

In the present invention, the stem cells may be at least one selected from the group consisting of bone marrow-derived mesenchymal stem cells, adipose-derived mesenchymal stem cells, embryonic stem cell-derived mesenchymal stem cells, tonsil-derived mesenchymal stem cells, and induced pluripotent stem cell-derived mesenchymal stem cells, but are not limited thereto. According to one experimental example of the present invention, the tissue regeneration effect of a stem cell spheroid prepared using embryonic stem cell-derived mesenchymal stem cells was confirmed, but the effect of the stem cell spheroid is not limited to the experimental example of the present invention, and the same effect may be exhibited in other types of stem cells described above.

In the present invention, the tissue may be one or more selected from the group consisting of skin, fat, salivary gland, trachea, airway, vocal cord, and muscle, and the muscle may be a hindlimb muscle, but is not limited thereto.

In the present invention, a "cell spheroid" refers to a cell cultured in a three-dimensional spherical form, and since cells in the body inherently have a three-dimensional structure, cell growth in spheroid form is superior and the original characteristics of the cells are better maintained compared to cells cultured in a monolayer. It also refers to a three-dimensional cell aggregate in which a hypoxic environment is induced due to limited oxygen delivery to the center of the spheroid, thereby increasing the expression of factors exhibiting various pharmacological activities. The cell spheroid of the present invention exhibits increased secretion of the above-mentioned pharmacologically active factors, that is, tissue regeneration-promoting factors known to promote tissue regeneration, including vascular endothelial growth factors (VEGFs), matrix metalloproteinases (MMPs), fibroblast growth factors (FGFs), epidermal growth factors (EGFs), hepatocyte growth factor (HGF), platelet-derived growth factors (PDGFs), placental growth factor (PIGF), granulocyte-macrophage colony-stimulating factor (GM-CSF), transforming growth factor beta (TGF-$\beta$), or angiopoietin, which are cytokines necessary for cell growth and differentiation, as well as immune-related cytokines such as the interleukin family. In particular, among these, VEGF is known to promote angiogenesis and recruit cytokines and immune cells necessary for tissue regeneration to the damaged tissue, thereby promoting tissue regeneration. Therefore, the cell spheroid in which the expression of these factors is enhanced according to the present invention may be effectively used for the treatment of various diseases requiring tissue regeneration.

In the present invention, the stem cell spheroid may be prepared by a method comprising the steps of: measuring the diameter of the stem cell; dispensing, into a culture vessel, preferably $1 \times 10^3$ to $1 \times 10^{10}$ stem cells, more preferably $3 \times 10^3$ to $3 \times 10^6$ stem cells, when the diameter of the stem cell is preferably 1 to 50 $\mu$m, more preferably 3 to 30 $\mu$m; and culturing the stem cells to obtain the stem cell spheroid.

In this case, the cells may be dispensed into the culture vessel in an amount corresponding to the required number of initial single cells (f) calculated by the following equation.

$$f = a \cdot exp^{-0.5\left[\left(\frac{x-x_0}{b}\right)^2 + \left(\frac{y-y_0}{c}\right)^2\right]}$$

In the above equation, f represents the required number of initial single cells, x represents the diameter of the single cell, and y represents the diameter of the cell spheroid. Preferably, xo may be from 8.2775 to 10.3231, yo may be from 624.8576 to 726.3632, a may be from 53.0017 to 89.4827, b may be from 9.0232 to 10.2778, and c may be from 162.0536 to 191.5685. More preferably, xo is from 7 to 11, yo is from 650 to 700, a is from 50 to 90, b is from 7 to 11, and c is from 150 to 200, but is not limited thereto.

By manufacturing the stem cell spheroid using the above method and function, a stem cell spheroid having a desired diameter can be uniformly produced.

In the present invention, the culturing step may involve culturing the cells preferably for 2 to 7 days, more preferably for 2 to 4 days, but is not limited thereto.

In the present invention, the composition may be in the form of a pharmaceutical composition, but is not limited thereto.

In the present invention, the composition may be used as a pharmaceutical composition for the prevention or treatment of a disease requiring tissue regeneration, but is not limited thereto.

Accordingly, the present invention provides a pharmaceutical composition for the prevention or treatment of a disease requiring tissue regeneration, including stem cell spheroids as an active ingredient.

In the present invention, the disease requiring tissue regeneration collectively refers to all diseases in which tissue regeneration is needed due to aging, damage, necrosis, or the like, and may include, for example, all diseases requiring regeneration in tissues such as bone, cartilage, skin, tympanic membrane, lung, fat, cartilage, bone, nerve, ligament, tendon, vocal cord, heart, liver, uterus, salivary gland, trachea, airway, muscle, and esophagus, but is not limited thereto. The stem cell spheroid may also be used for regenerating damaged tissue in ischemic diseases. Alternatively, it may be used for tissue regeneration after tissue resection surgeries such as cancer excision surgery. However, it is not limited thereto as long as the disease can be treated by regenerating tissue using stem cells.

In the present invention, "ischemia" refers to a state in which a blood vessel supplying blood to an organ, tissue, or body part becomes narrowed or constricted, or normal vascular formation is insufficient, resulting in impaired blood supply and localized tissue necrosis. In particular, the heart and brain are organs most sensitive to insufficient blood supply, and when ischemia occurs in the tissue, a series of processes called the ischemic cascade is triggered, leading to permanent tissue damage. A disease exhibiting such ischemic symptoms is referred to as an "ischemic disease." The ischemic diseases may include ischemic hindlimb disease, angina pectoris, myocardial infarction, cerebral infarction, and foot ulcer, and the ischemic hindlimb diseases may include peripheral arterial occlusive disease, peripheral arterial stenotic disease, and peripheral arterial sclerosis.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel®; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite ($NaHSO_3$) carbon dioxide gas, sodium metabisulfite ($Na_2S_2O_5$), sodium sulfite ($Na_2SO_3$), nitrogen gas ($N_2$), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro (OSI, OSIX, A, B, C, D, H, L), suppository base IV types (AB, B, A, BC, BBG, E, BGF, C, D, 299), suppostal (N, Es), Wecoby (W, R, S, M, Fs), and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered *via*, for example, oral ingestion, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, direct implantation into a wound site or the like.

The pharmaceutical composition of the present invention is determined depending on the type of a drug, which is an active ingredient, along with various related factors such as a disease to be treated, administration route, the age, gender, and body weight of a patient, and the severity of diseases.

In the present invention, the composition may further include a scaffold for tissue engineering, but is not limited thereto. Scaffolds for tissue engineering have been developed to replace defect sites in the event of tissue defects such as burns or cancer tissue removal. However, when a scaffold for tissue engineering is used alone, the speed of tissue regeneration is significantly delayed, and there are limitations in tissue regeneration due to side effects caused by immune rejection such as inflammation. In the present invention, it was confirmed that when the stem cell spheroid is used in combination with a scaffold for tissue engineering, the efficacy of tissue regeneration is enhanced.

In the present invention, the scaffold for tissue engineering may be implanted through surgical transplantation or by using a syringe or the like, and may include biocompatible or biodegradable polymers including poly(caprolactone) (PCL) or the like, or hydrogels, but is not limited thereto.

In the present invention, the composition may further include a scaffold for tissue engineering and two-dimensionally cultured stem cells, but is not limited thereto. According to one experimental example of the present invention, the scaffold may be coated with two-dimensionally cultured stem cells on the inside and coated with stem cell spheroids on the outside, but is not limited thereto.

The present invention provides a composition or a kit for promoting tissue regeneration, comprising a scaffold for tissue engineering, wherein two-dimensionally cultured stem cells are coated on the inside and stem cell spheroids are coated on the outside.

In the present invention, the "kit" refers to a tool that promotes tissue regeneration by comprising a scaffold for tissue engineering coated with the two-dimensionally cultured stem cells and the stem cell spheroids. The kit of the present invention may further include other components, compositions, solutions, devices, and the like that are conventionally required, in addition to the above formulation. In the present invention, the kit may include a container, an instruction manual, and the scaffold. The container may serve to package the scaffold, and may also serve to store and secure it. The material of the container may take the form of, for example, a bottle, tub, sachet, envelope, tube, or ampoule, and may be formed partially or entirely from plastic, glass, paper, foil, wax, or the like. The container may be equipped with a cap that is initially a part of the container or is completely or partially detachable and attachable to the container by mechanical, adhesive, or other means, and may also be equipped with a stopper that allows access to the contents with a syringe needle. The kit may include an external package, and the external package may include instructions for use of the components.

In the present invention, the stem cell spheroids may exhibit enhanced expression of bioactive factors associated with promotion of tissue regeneration, but is not limited thereto.

In the present invention, the bioactive factors may be one or more selected from the group consisting of vascular endothelial growth factors (VEGFs), matrix metalloproteinases (MMPs), fibroblast growth factors (FGFs), epidermal growth factors (EGFs), hepatocyte growth factor (HGF), platelet-derived growth factor (PDGF), placental growth factor (PlGF), granulocyte-macrophage colony-stimulating factor (GM-CSF), transforming growth factor beta (TGF-β), and angiopoietin, but are not limited thereto.

In the present invention, the stem cells may promote angiogenesis, but are not limited thereto.

In the present invention, "angiogenesis" refers to a physiological process in which new blood vessels are formed from existing blood vessels, and occurs at an early stage of vasculogenesis. Angiogenesis mainly sustains the growth of vascular structures through the processes of sprouting and branching, but processes such as intussusceptive angiogenesis, vascular elongation, and vascular fusion also play a role. Angiogenesis is a normal and important process in growth and development, wound healing, and granulation tissue formation.

In the present invention, the stem cell spheroid may effectively promote tissue regeneration by inducing a hypoxic environment inside, thereby promoting the secretion of various pharmacologically active factors such as angiogenic factors and cell growth factors, but is not limited thereto.

In the present invention, the cell spheroids may have a diameter of 100 to 500 $\mu$m, 100 to 450 $\mu$m, 100 to 400 $\mu$m, 100 to 350 $\mu$m, 100 to 300 $\mu$m, 100 to 250 $\mu$m, 100 to 200 $\mu$m, 150 to 500 $\mu$m, 150 to 450 $\mu$m, 150 to 400 $\mu$m, 150 to 350 $\mu$m, 150 to 300 $\mu$m, 150 to 250 $\mu$m, 150 to 200 $\mu$m, 200 to 500 $\mu$m, 200 to 450 $\mu$m, 200 to 400 $\mu$m, 200 to 350 $\mu$m, 200 to 300 $\mu$m, 200 to 250 $\mu$m, 250 to 500 $\mu$m, 250 to 450 $\mu$m, 250 to 400 $\mu$m, 250 to 350 $\mu$m, 250 to 300 $\mu$m, 200 $\mu$m, 250 $\mu$m, 300 $\mu$m, 350 $\mu$m, or 400 $\mu$m, but is not limited thereto.

Additionally, the present invention provides a cell therapeutic agent for promoting tissue regeneration, comprising stem cell spheroids as an active ingredient.

In the present invention, "cell therapeutic agent (cell therapy)" refers to a pharmaceutical product (as defined by the United States FDA regulations) that is a stem cell spheroid prepared using stem cells isolated, cultured, and specially manipulated from mammals, and is used for therapeutic, diagnostic, and preventive purposes, and refers to a pharmaceutical product used for therapeutic, diagnostic, and preventive purposes through a series of actions such as proliferating, selecting, or otherwise modifying the biological characteristics of homologous or heterologous cells *ex vivo* in order to restore the function of cells or tissues. The cell therapy agent of the present invention may further include one or more active ingredients exhibiting the same or similar functions in addition to the stem cell spheroid. Additionally, for administration, it may be prepared by further including one or more pharmaceutically acceptable carriers. The pharmaceutically acceptable carrier may include binders, lubricants, disintegrants, diluents, solubilizers, dispersants, stabilizers, suspending agents, colorants, and flavors for oral administration; may include buffers, preservatives, analgesics, solubilizers, isotonic agents, and stabilizers for injectable administration; and may include bases, diluents, lubricants, and preservatives for topical administration. The formulation of the cell therapy agent of the present invention may be variously prepared by mixing with the pharmaceutically acceptable carrier as described above. For example, in the case of oral administration, it may be prepared in the form of a tablet, troche, capsule, elixir, suspension, syrup, or wafer, and in the case of injectable administration, it may be prepared in the form of a unit dose ampoule or a multiple dose form; and additionally, it may be formulated as a solution, suspension, tablet, capsule, or sustained-release preparation.

The administration route of the cell therapy agent according to the present invention is not limited thereto, but includes oral, intravenous, intramuscular, intraarterial, intra-bone marrow, intradural, intracardiac, transdermal, subcutaneous, intraperitoneal, intranasal, intestinal, local, sublingual, or rectal administration. Oral or parenteral administration is preferable. As used herein, the term "parenteral" includes subcutaneous, intradermal, intravenous, intramuscular, intra-articular, intrasynovial, intrasternum, intradural, intralesional, and intracranial injection or infusion techniques.

The stem cell therapy agent of the present invention may vary depending on various factors including the activity, age, weight, general health, gender, diet, time of administration, route of administration, excretion rate, drug combination, and the severity of the particular disease to be prevented or treated, and may be appropriately selected by those skilled in the art. For example, the stem cell spheroid may be administered in an amount of 10 to 18,000, 100 to 18,000, 500 to 18,000, 1,000 to 18,000, 5,000 to 18,000, 10,000 to 18,000, 15,000 to 18,000, 15,500 to 18,000, 16,000 to 18,000, 15,000 to 17,000, 15,500 to 17,000, 16,000 to 17,000, 15,000 to 16,000, 15,500 to 16,000, or 16,000 stem cell spheroids. The administration may be performed once a day or divided into several doses per day. Additionally, when formulated into a liquid unit dosage form such as a solution, suspension, or emulsion, it may also be administered to a patient at the above-described cell concentration.

Additionally, the present invention provides a method for promoting tissue regeneration, comprising administering a composition comprising stem cell spheroids as an active ingredient to a subject in need thereof.

Additionally, the present invention provides a use of a composition comprising stem cell spheroids as an active ingredient for the promotion of tissue regeneration.

Additionally, the present invention provides a use of a composition comprising stem cell spheroids as an active ingredient for the preparation of a formulation for promoting tissue regeneration.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby *via* administration of the pharmaceutical composition according to the present invention.

In the present invention, when the term "comprising" is used, it does not exclude other components unless otherwise specified, but means that other components may be further included. Throughout the present invention, the terms "step of ~" or "the step of ~" do not mean "step for ~."

Hereinafter, preferred examples are presented to help understand the present invention. However, the following examples are provided only to help understand the present invention more easily, and the contents of the present invention are not limited by the following examples.

## [Examples]

### Example 1. Fabrication of stem cell spheroids

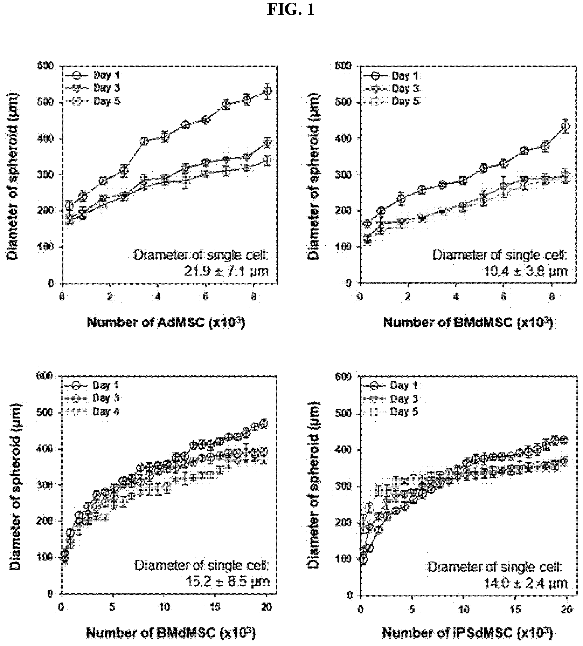

To fabricate stem cell spheroids, first, each type of bone marrow-derived mesenchymal stem cells (BM-MSCs), adipose-derived mesenchymal stem cells (AD-MSCs), and induced pluripotent-derived mesenchymal stem cells (iPS-MSCs), which were grown in monolayers, were isolated as single cells by trypsin/EDTA treatment. Each type of the isolated single cells were then prepared at various concentrations, seeded in 50 $\mu$L aliquots along with culture medium into an agar mold with 35 wells of a specific size and cultured under 37 °C and 5% $CO_2$ conditions. The initial number of stem cells used in culture was 1 x $10^4$ to 30 x $10^4$ cells/well for AD-MSCs, or 1 x $10^4$ to 69 x $10^4$ cells/well for BM-MSCs and iPS-MSCs. In addition, the diameter of a stem cell spheroid according to the number of single cells was measured on days 1, 3, and 5. The results are shown in FIG. 1.

As shown in FIG. 1, 35 stem cell spheroids were fabricated, and the diameters of the stem cell spheroids on day 1 were greater than those on days 3 and 5, showing a decreasing trend over the culture period. Through the above results, a decrease in diameters of stem cell spheroids was observed over time as reactions such as cell-cell interactions or cell-ECM interactions progressed, and this change in diameter was prominently observed during the early stages of spheroid formation, but became minimal afterward.

### Example 2: Measurement of required number of single cells according to diameter of stem cell spheroid

To measure the required number of single cells according to the diameter of a stem cell spheroid, the diameter of stem cell spheroids formed on day 3 was measured, the relationship between the initial number of single cells and single-cell diameter in each type of stem cell spheroids having diameters of 150 $\pm$ 10 $\mu$m, 200 $\pm$ 10 $\mu$m, and 250 $\pm$ 10 $\mu$m, respectively, was analyzed

through logistic regression (Polynominal, Linear regression, SigmaPlot12.0), and a regression curve was drawn.

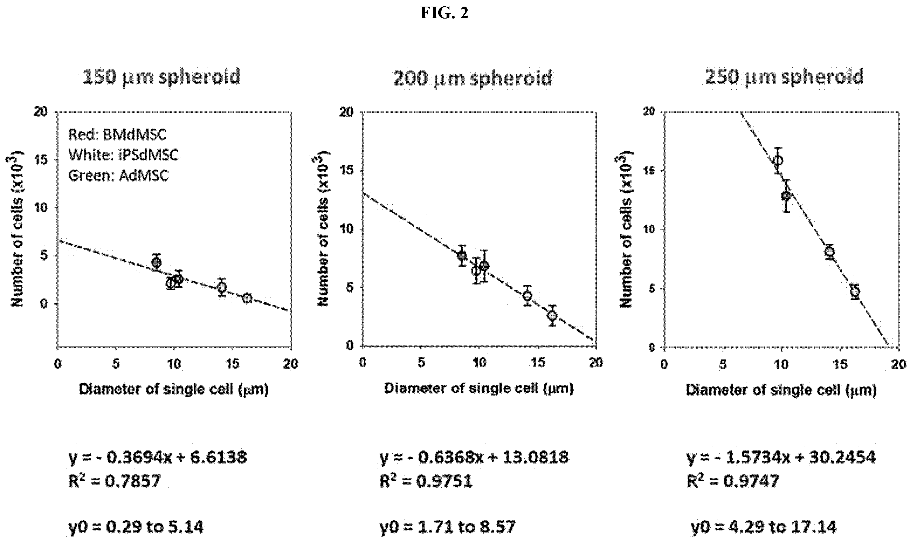

The diameter of single cells was 10.4 $\pm$ 3.8 $\mu$m and 15.2 $\pm$ 8.5 $\mu$m for BM-MSCs, 14.0 $\pm$ 2.4 $\mu$m for iPS-MSCs, and 21.9 $\pm$ 7.1 $\mu$m for AD-MSCs. The results are shown in FIG. 2.

As shown in FIG. 2, it was confirmed that, using the diameter of each type of single cells, the number of single cells required to fabricate a cell spheroid of a desired diameter can be calculated from the regression curve.

In addition, the above results were presented as a 3D graph using a rotation transformation matrix, and the correlation between single-cell diameter, cell spheroid diameter, and the number of single cells was expressed as a function. The results are shown in FIG. 3.

As shown in FIG. 3, the function of calculating the initial required number of single cells required to fabricate a cell spheroid of a desired diameter is as follows:

$$f = a \cdot exp^{-0.5\left[\left(\frac{x-x_0}{b}\right)^2 + \left(\frac{y-y_0}{c}\right)^2\right]}$$

In the above function, f is the initial required number of single cells, x is the diameter of single cells, y is the diameter of a cell spheroid, and it was confirmed that, when $x_0$ is 8.2775 to 10.3231, $y_0$ is 624.5876 to 726.3632, a = 53.0017 to 89.4827, b = 9.0232 to 10.2778, and c = 162.0563 to 191.5685, a significant result is obtained, and especially, when $x_0$ = 9.3003, $y_0$ = 675.4754, a = 71.2422, b = 9.6505, and c = 176.8124, $R^2$ = 0.9631. Therefore, it was confirmed that the initial required number of single cells to fabricate a cell spheroid of a desired diameter can be quantified by the above function. In addition, regardless of the type of stem cells, the diameter of single cells serves as a critical factor for determining the spheroid diameter.

### Example 3: Verification of method of measuring required number of single cells according to diameter of stem cell spheroid

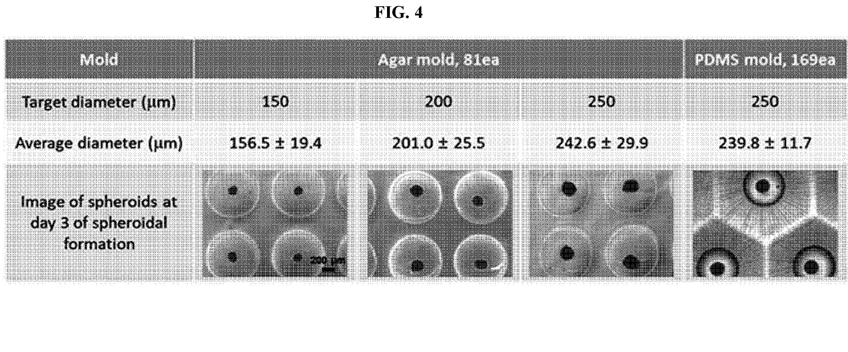

To verify whether the method of measuring a required number of single cells according to the present invention can be applied to various culture vessels, experiments were conducted using culture vessels with various sizes and shapes. In more detail, the required number of AD-MSC single cells were seeded for forming a spheroid of a desired diameter into an agar mold with 81 circular wells of uniform size or a PDMS mold with 169 hexagonal wells of uniform size and cultured for 3 days to examine whether stem cell spheroids of a desired diameter were produced. The results are shown in FIG. 4.

As shown in FIG. 4, it was confirmed that the stem cell spheroids of a desired diameter were fabricated regardless of the type of culture vessel. Through the above result, it was confirmed that the function of calculating the initial required number of single cells according to the present invention can be applied regardless of the size and shape of a culture vessel.

## Example 4. Characterization of stem cell spheroid properties

### 4-1. Confirmation of induction of hypoxic environment according to diameter of stem cell spheroid

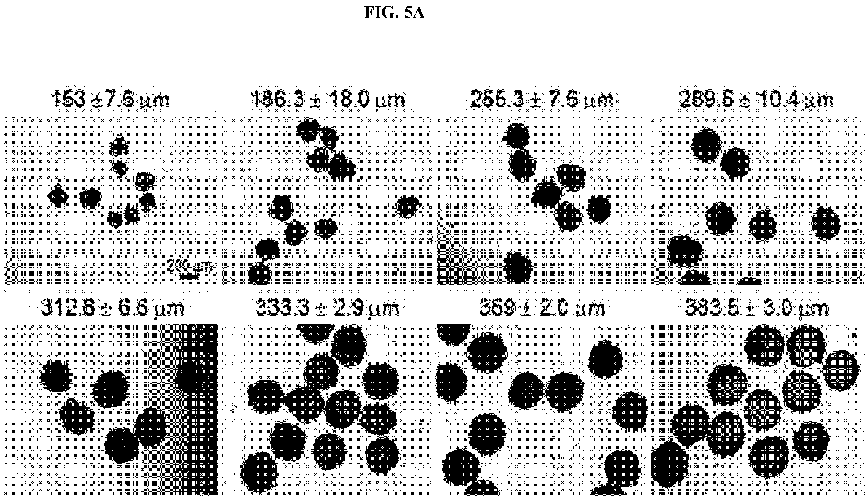

To confirm the degree of a hypoxic environment induced within a stem cell spheroid according to the spheroid diameter, the AD-MSC spheroids on day 1 were treated with lectin-like oxidized low-density lipoprotein (LDL) receptor-1 (Lox-1), which is used to determine hypoxic conditions, and further cultured for one day. In addition, the hypoxic degree was observed under a fluorescence microscope. The results are shown in FIG. 5A.

As shown in FIG. 5A, it was confirmed that no red fluorescence was observed in the stem cell spheroid with a diameter of 186 $\mu$m, indicating that hypoxic conditions were not created, but red fluorescence was observed in the stem cell spheroids with diameters of 200 $\mu$m or more. Through the above results, it can be demonstrated that the hypoxic environment was induced within the stem cell spheroids with diameters of 200 $\mu$m or more, and therefore, the expression level of bioactive factors expressed in the hypoxic environment would increase.

### 4-2. Evaluation of cell viability according to diameter of stem cell spheroid

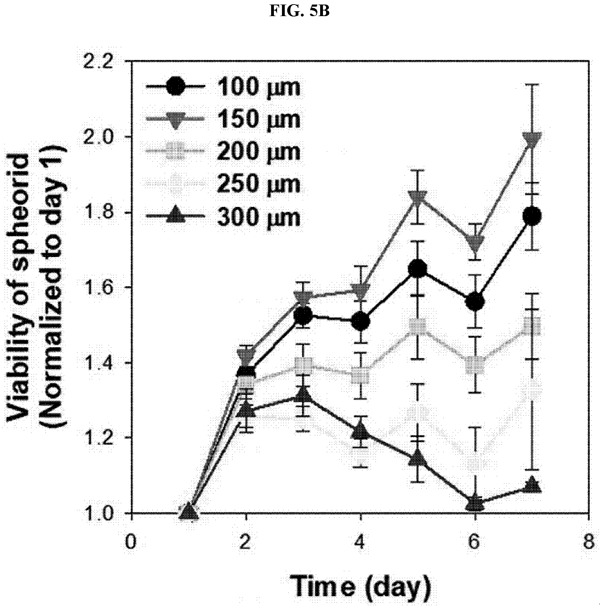

To evaluate cell viability according to the diameter of a stem cell spheroid, stem cell spheroids with a diameter of 100 to 300 $\mu$m were fabricated from embryonic stem cell-derived mesenchymal stem cells using the function of calculating the initial required number of single cells required to fabricate a stem cell spheroid of a desired diameter, which had been established in Example 2, and then the spheroids were further cultured for 7 days to evaluate cell viability using Alamar Blue assay. The results are shown in FIG. 5B.

As shown in FIG. 5B, cell viability was reduced in cell spheroids with diameters of 250 $\mu$m or more, suggesting a hypoxic environment induced within the spheroids and a subsequent increase in cell death.

### 4-3. Evaluation of degree of bioactive factor secretion according to diameter of stem cell spheroid

To evaluate the degree of bioactive factor secretion according to the diameter of stem cell spheroid, each type of single cell dispersion (50 $\mu$L aliquots) was dispensed into an agar mold with 35 wells of uniform size, 450 $\mu$L of culture medium was additionally added into each well, and then the cells were cultured under 37 °C

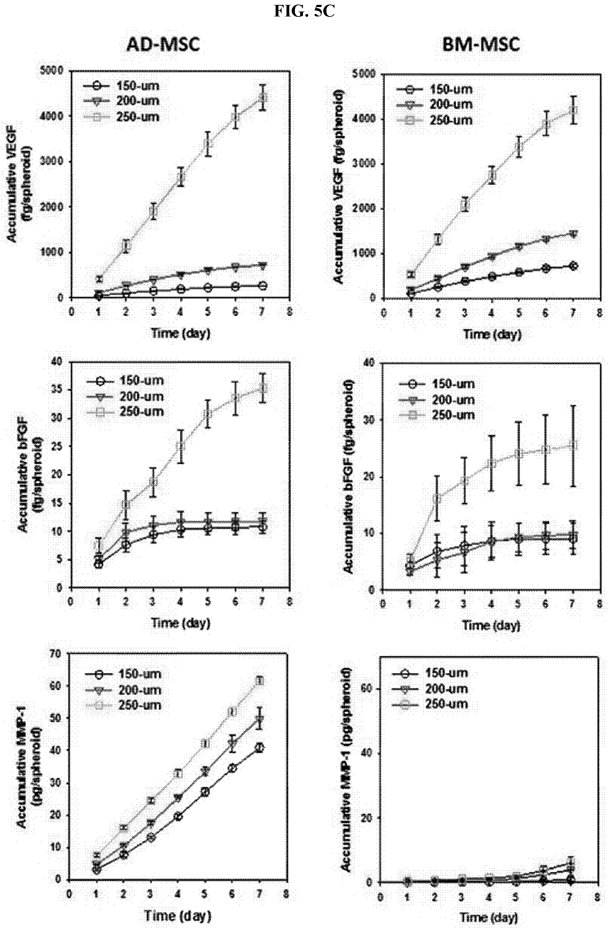

and 5% $CO_2$ conditions. To ensure that the diameter of each stem cell spheroid reached 150 $\mu$m on day 3 of culture, the initial number of stem cells used in the culture was 1.71 x $10^3$ cells per well for AD-MSCs, or 2.57 x $10^3$ cells per well for BM-MSCs; to ensure that the diameter of each stem cell spheroid reached 200 $\mu$m on day 3 of the culture, the initial number of stem cells used in culture was 4.28 x $10^3$ cells per well for ADMSCs, or 6.00 x $10^3$ cells per well for BM-MSCs; and to ensure that the diameter of each stem cell spheroid reached 250 $\mu$m on day 3 of the culture, the initial number of stem cells used in culture was 8.57 x $10^3$ cells per well for AD-MSCs, or 12.86 x $10^3$ cells per well for BM-MSCs. In addition, the culture broth obtained by culturing for 24 hours while replacing the culture medium daily with fresh medium was collected to measure the amount of secreted bioactive factors using ELISA. The bioactive factors measured in the experiment were representatively known vascular endothelial growth factor (VEGF), basic fibroblast growth factor (bFGF), and matrix metalloproteinase-1 (MMP-1). For each mold, 35 stem cell spheroids were maintained, and a medium amount was maintained at 500 $\mu$L. The cumulative secretion amount of a pharmacologically active factor is shown in FIG. 5C.

As shown in FIG. 5C, as the diameter of a stem cell spheroid increased, the expression level of a bioactive factor increased, and it was confirmed that the expression level of a bioactive factor was the highest when the diameter of a stem cell spheroid was 250 $\mu$m. More specifically, it was confirmed that VEGF, which is known to induce neovascularization, increased as diameters increased in both AD-MSCs and BM-MSCs, and particularly, the expression level in stem cell spheroids with a diameter of 250 $\mu$m increased up to 8 times or more compared to that in stem cell spheroids with a diameter of 150 $\mu$m. In addition, it was confirmed that bFGF, which is known to promote cell growth, also exhibits the highest expression level in the stem cell spheroids with a diameter of 250 $\mu$m. It was confirmed that the expression level of MMP-1, which induces cell migration by destroying the extracellular matrix, was low in BM-MSCs, and the stem cell spheroid with a diameter of 250 $\mu$m exhibited the highest expression level. It was confirmed that, for AD-MSCs, the cell spheroid with a diameter of 250 $\mu$m exhibited the highest expression level. Through the above results, it was confirmed that the expression level of a bioactive factor was increased in stem cell spheroids with diameters of 200 $\mu$m or more, in which the hypoxic environment was induced within the cells, and therefore it was expected that the stem cell spheroids with diameters of 200 to 400 $\mu$m would have a high tissue regeneration effect.

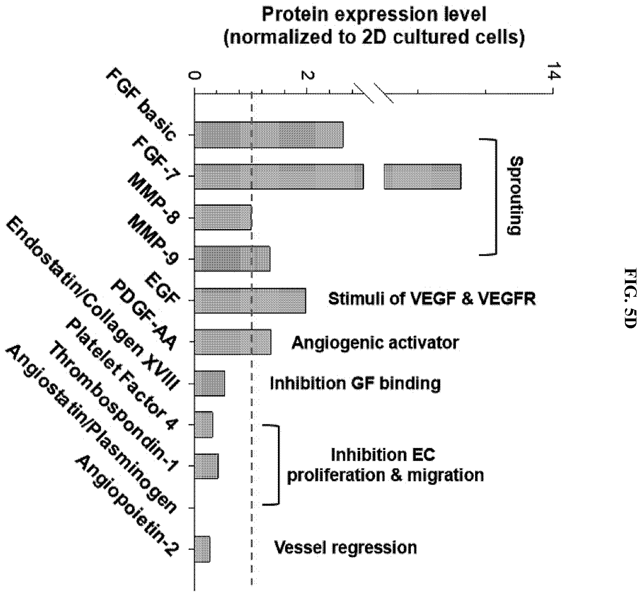

Furthermore, compared to 2D-cultured stem cells, bioactive factors expressed in stem cell spheroids were quantified. A culture broth obtained three days after stem cell spheroids were prepared in the same manner as in Examples 1 and 2 using embryonic stem cell-derived mesenchymal stem cells was collected to confirm various bioactive factors involved in the process of angiogenesis using the proteome profiler human angiogenesis array kit (R&D System). As a result, as shown in FIG. 5D, it was demonstrated that the expression levels of angiogenesis-inducing factors were increased in stem cell spheroids compared to single cells.

## [Experimental Examples]

## Experimental Example 1. Confirmation of expression of angiogenesis promoting factor (VEGF) in stem cell spheroid and verification thereof

To verify whether VEGF among various cytokines highly expressed in stem cell spheroids affects angiogenesis, the tubule formation of green fluorescent protein-expressing human umbilical vein endothelial cells (GFP-HUVECs) by stem cell spheroids was examined using a transwell system.

In more detail, to examine tubule formation by VEGF secreted from stem cell spheroids, a 24-well plate was coated with a Matrigel® matrix without growth factors, and HUVECs were seeded at $2.6 \times 10^3$ cells/cm$^2$. In addition, 20 stem cell spheroids were seeded on an upper part of the plate using a transwell system, and cultured for 6 to 24 hours. Here, the stem cell spheroids were fabricated in the same manner as in Examples 1 and 2 using embryonic stem cell-derived mesenchymal stem cells, and very uniformly produced. The stem cell spheroids used here were spheroids with a diameter of 300 $\mu$m, which were collected from the culture on day 3. After 6 and 24 hours of culture, master junctions and master segments were analyzed and quantified using ImageJ angiogenesis analysis.

Here, it was verified whether the presence or absence of anti-VEGF as an antagonist of VEGF expressed in stem cell spheroids affects the tubule formation of GFP-HUVEC. Since a culture medium without VEGF was used, VEGF affecting GFP-HUVEC was identified as being specifically expressed by stem cell spheroids. Anti-VEGF was used at half (0.53 pM) or an equal amount (1.07 pM) compared to the amount expressed by the 20 stem cell spheroids with a diameter of 300 $\mu$m.

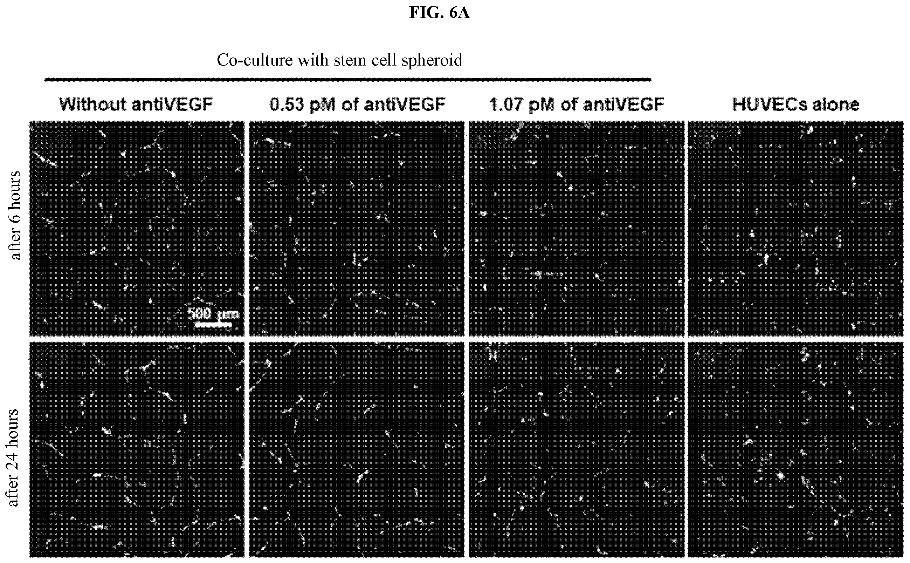

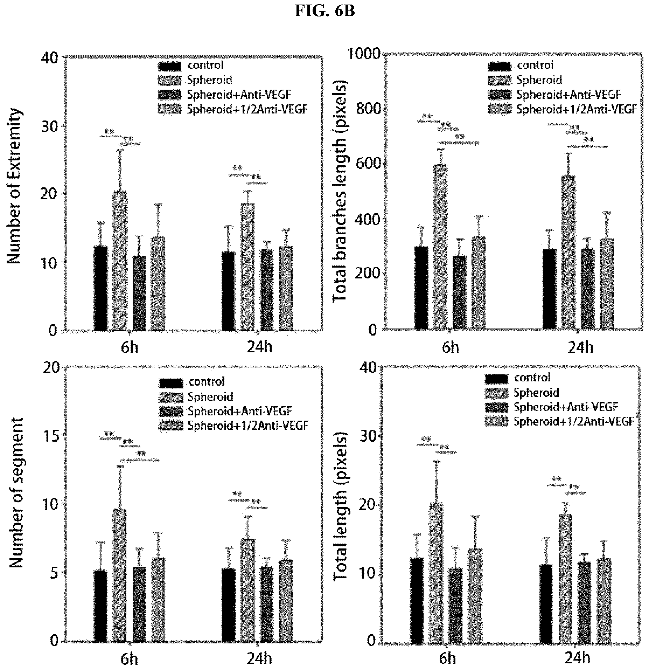

As a result, as shown in FIG. 6A, it was confirmed that, compared to a control in which HUVECs were cultured without stem cell spheroids, when HUVECs were cultured along with stem cell spheroids, the tubule formation of GFP-HUVECs active progressed, and anti-VEGF treatment significantly reduced the degree of tubule formation. In addition, as shown in FIG. 6B, the quantitative results also showed that tubule formation increased when co-cultured with stem cell spheroids, and that the increasing trend of tubule formation was attenuated in the experimental group treated with anti-VEGF. Therefore, it was demonstrated that VEGF expressed in stem cell spheroids affects the angiogenesis of vascular endothelial cells.

## Experimental Example 2. Verification of efficacy in preventing salivary gland damage caused by radiation exposure

To confirm the efficacy of stem cell spheroids in preventing salivary gland dysfunction induced by radiation exposure, an oral xerostomia animal model with solid oral cancer was established and verified. For non-clinical efficacy evaluation, aging mice (Balb.c/nude, 12 months, male) were used, and to induce solid cancer in the mouth, $5 \times 10^5$ SNU-1041 cells were dispersed in 20 $\mu$L of PBS and injected into the right side of the tongue of the anesthetized mouse using a 27G insulin syringe. As an anesthetic, zoletile/rampun was used in a 1:3 dilution. One week after injection, the anesthetized mouse was placed in the supine position and then treated with a single dose of 15 Gy of radiation exposure using a 21EX-S device to the area including the salivary glands and tongue. 48 hours after radiation exposure, the anesthetized mouse was placed in the supine position, a 1-cm incision was made under the skin of the jaw to expose the salivary gland tissue, and 81 embryonic stem cell-derived mesenchymal stem cell spheroids were injected using a 23G needle. Here, the stem cell spheroids were dispersed in 20 $\mu$L of saline. The body weight of the mouse and a change in the size of solid cancer were monitored at one-week intervals, and 6 weeks after stem cell spheroid transplantation, the body weight of the mouse decreased by more than 20% compared to the initial weight, so it was sacrificed in accordance with the Animal Ethics Act. Then, the salivary gland tissue was recovered and analyzed using paraffin block sections to observe histopathological changes.

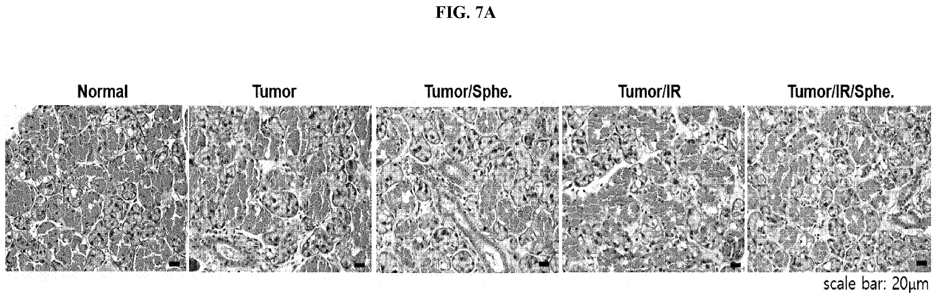

As a result of histopathological evaluation by H&E staining, as shown in FIG. 7A, compared to the salivary tissue (Normal) of the aging mouse, oral cancer (Tumor), spheroid injection (Tumor/Sphe., Tumor/-IR/Sphe.), and radiation exposure (Tumor/IR) did not affect the morphology of the salivary gland tissue.

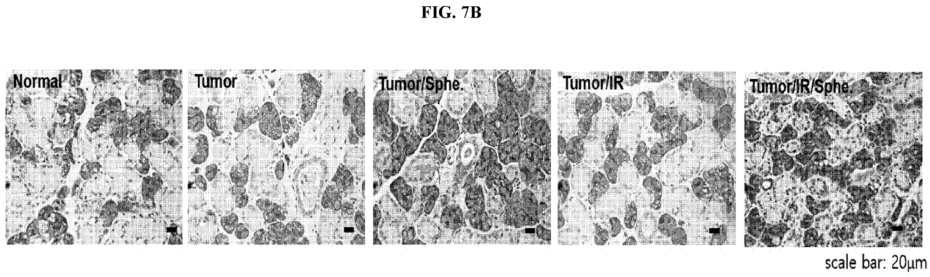

In addition, as a result of confirming the mucin secretion function of the salivary glands through Alcian blue staining (mucin: blue), as shown in FIG. 7B, it was confirmed that the mucin secretion function as reduced in the salivary glands of the oral cancer (tumor) model compared to the normal salivary glands, whereas the mucin secretion function was improved in the spheroid-injected group (Tumor/Sphe.). Particularly, in the case of the salivary glands of the oral cancer model (Tumor/IR) that was exposed to radiation, the tumor was initially suppressed by radiation exposure, but the mucin secretion function was reduced due to the damaged salivary glands caused by radiation exposure. However, in the case of injecting stem cell spheroids (Tumor/IR/Sphe.), it was confirmed that the mucin secretion function was significantly improved by the injection of stem cell spheroids even when the salivary glands were exposed to radiation.

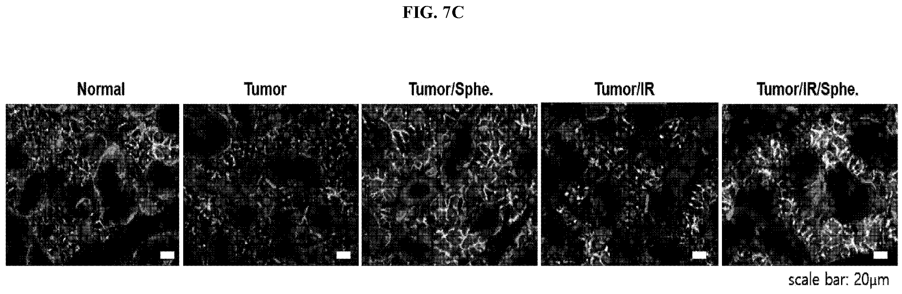

In addition, to evaluate the secretory function and morphology of the salivary glands, immunochemical staining was used to stain the acini constituting the salivary glands with aquaporin 5 (APQ5: cyanine color), which is a protein expressed in the acini, and the myoepithelial cells surrounding them were stained with alpha-smooth muscle actin (α-SMA: red) and observed. As a result, as shown in FIG. 7C, it was confirmed that APQ5 and myoepithelial cells were damaged by tumors and radiation exposure, and in the case of salivary glands transplanted with stem cell spheroids, damage was prevented and the damaged tissue was regenerated. In addition, in the Normal group, AQP5 expression levels in the salivary glands of the mice were low due to aging, whereas the expression levels increased when stem cell spheroids were transplanted, confirming that the stem cell spheroids are also effective in restoring the functional decline of the salivary glands due to aging.

### Experimental Example 3. Verification of efficacy in reconstructing partial tracheal defects

#### 3-1. Confirmation of results of tracheal scaffold transplantation

To evaluate the efficacy of stem cell spheroids in recovering from tracheal defect diseases, a poly(caprolactone) (PCL) tracheal scaffold with a surface of a leaf-stacked structure was randomly selected as a model scaffold for tissue engineering and used. For animal models, full tracheal defect or partial tracheal defect animal models were used.

First, a tracheal defect model was prepared by making a 5 mm x 9 mm defect in the trachea of a male New Zealand white rabbit. In addition, the defect site was sealed using a PCL nanofiber membrane (9 mm x 11.5 mm) as a tracheal scaffold.

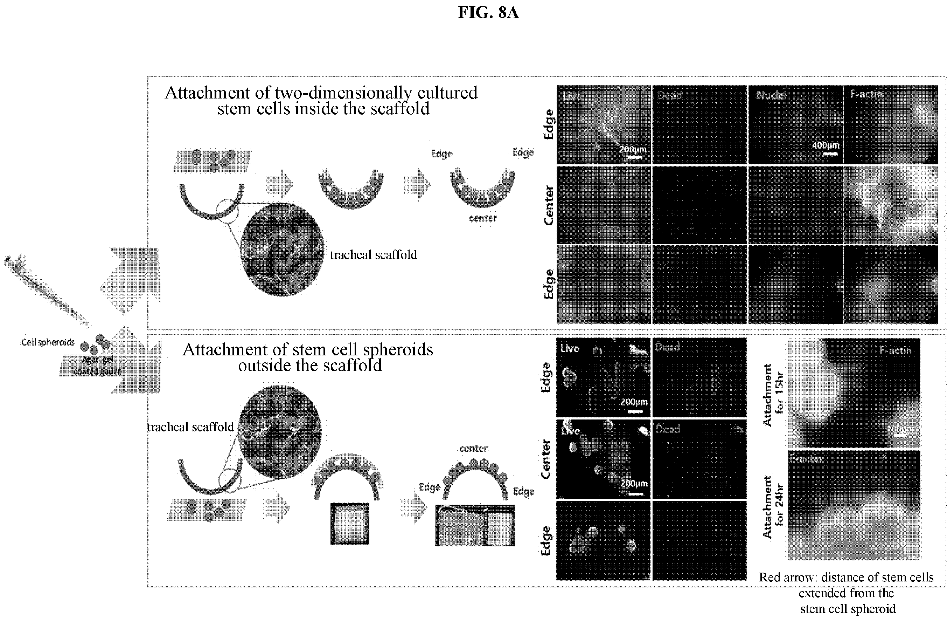

320 embryonic stem cell-derived mesenchymal stem cell spheroids were applied onto the outside of the scaffold considering the direction of the tracheoesophageal artery, 2D-cultured stem cells were applied at $1.6 \times 10^6$ cells/scaffold onto the inside of the scaffold to observe the regeneration of the airway mucosa and angiogenesis. By using gauze coated with agar gel to apply the 2D-cultured stem cells and stem cell spheroids to the tracheal scaffold, an environment was created so that the cells used could be completely attached to the scaffold. As a result, as shown in FIG. 8A, it was confirmed that all cells were attached to the tracheal scaffold regardless of the morphology of the stem cell, and had excellent viability.

In addition, the experiment was completed 14 weeks after transplanting the 2D-cultured stem cell- and stem cell spheroid-applied tracheal scaffolds for reconstructing partial tracheal defects into the partial tracheal defect sites of the rabbits, and all rabbits survived until the end of the experiment. The image illustrating the tracheal scaffold transplantation and the image of the tracheal scaffold 14 weeks after transplantation are shown in FIG.

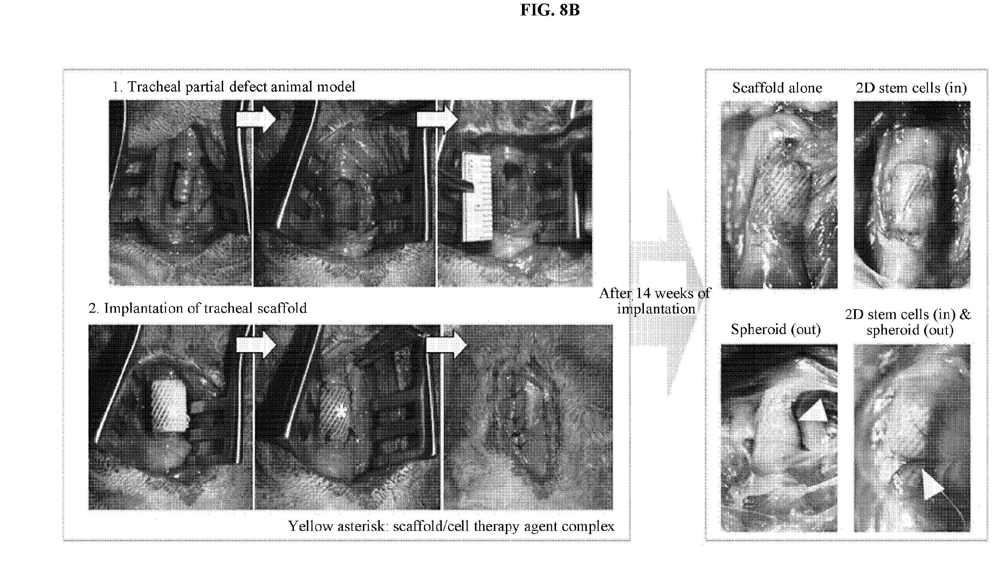

8B. As shown in FIG. 8B, 14 weeks after transplantation, in a tracheal scaffold to which the stem cell spheroids were not applied, no noticeable induction of angiogenesis was observed, whereas, in the experimental group to which the stem cell spheroid-coated scaffold was transplanted, it was confirmed that new blood vessels were formed from the surrounding thick blood vessels and entered the tracheal scaffold. It was confirmed that the new blood vessels formed into the transplanted tracheal scaffold were similar to blood vessels provided in the normal airway, and thus they would greatly assist tissue regeneration.

#### 3-2. Confirmation of airway mucosa regeneration through endoscopy

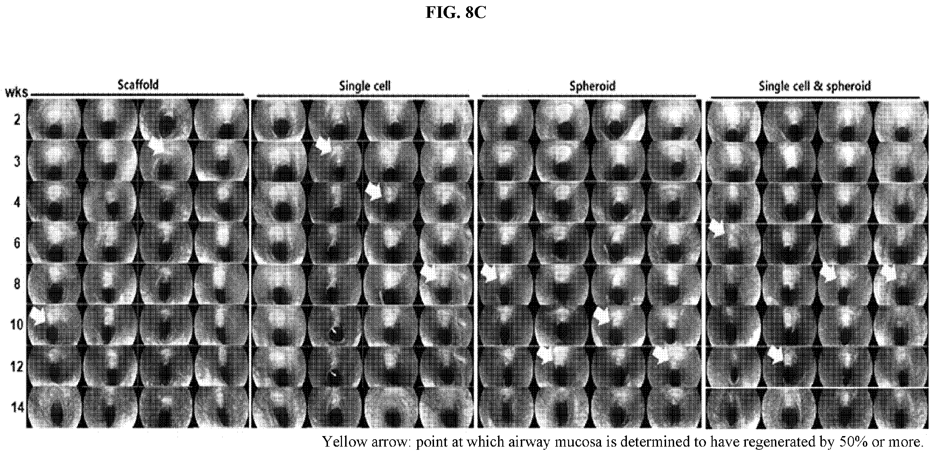

As a result of observing the endoscopic images for 14 weeks after tracheal scaffold transplantation, as shown in FIG. 8C, in the case of stem cell spheroid-introduced scaffolds, the airway mucosa was more than 50% regenerated in the airways of all rabbit models.

In addition, even when 2D stem cells were applied inside a tracheal scaffold, it was confirmed that the airway mucosa was rapidly regenerated regardless of angiogenesis and the airway patency was well secured. Considering the rabbit models used in the experiment had partial tracheal defects, it was assumed that the fusion of 2D stem cells applied inside the tracheal scaffold proceeded rapidly through the surrounding blood vessels because the original blood vessels connected to the trachea were not completely removed.

#### 3-3. Histological analysis results

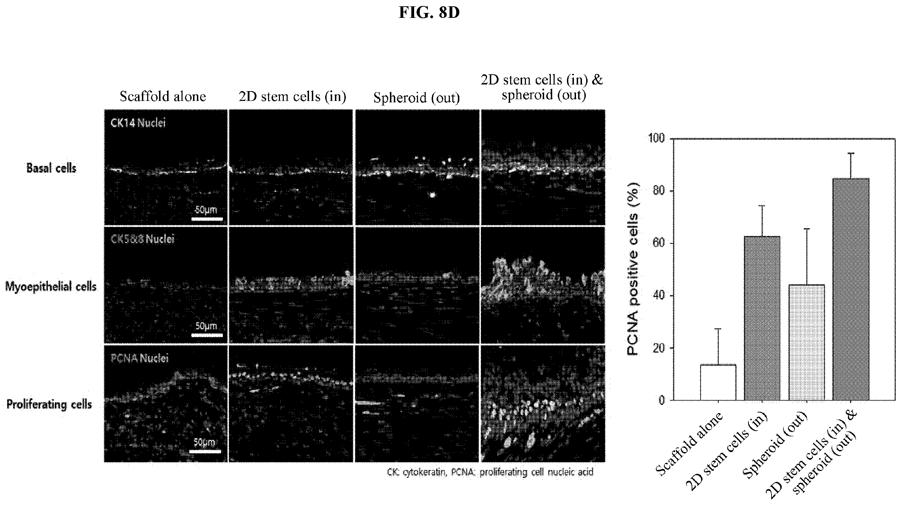

Histological analysis was performed by recovering tracheal tissue. As shown in FIG. 8D, according to the immunostaining of the tracheal tissue, basal cells were detected in all experimental groups, indicating the potential for regeneration into the airway mucosa. It was confirmed that, compared to the case where the scaffold alone was transplanted, when each of the 2D-cultured stem cells and the stem cell spheroids were applied, regenerative effects were exhibited. Particularly, when each of the 2D-cultured stem cells and the stem cell spheroids were applied to inside and outside the scaffold, it was confirmed that the airway mucosa layer was also regenerated. In addition, it was confirmed that when both 2D-cultured stem cells and stem cell spheroids were applied, more proliferating cells were found as compared to the other experimental groups. Accordingly, it was confirmed that a synergistic effect was achieved when both types of cells were used.

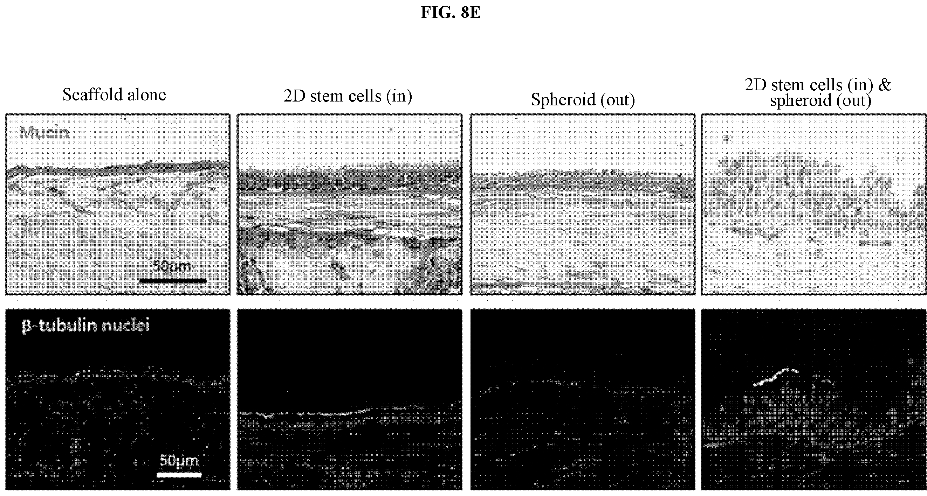

In addition, to confirm the functional regeneration of cells of the regenerated airway mucosa layer, as a result of confirming goblet cells secreting mucin through Alcian blue staining, goblet cells were clearly shown when 2D-cultured stem cells were applied inside the scaffold and when 2D-cultured stem cells and stem cell spheroids were applied inside and outside the scaffold, as shown in FIG. 8E. A similar trend was observed in beta-tubulin immunostaining to confirm cilia formation.

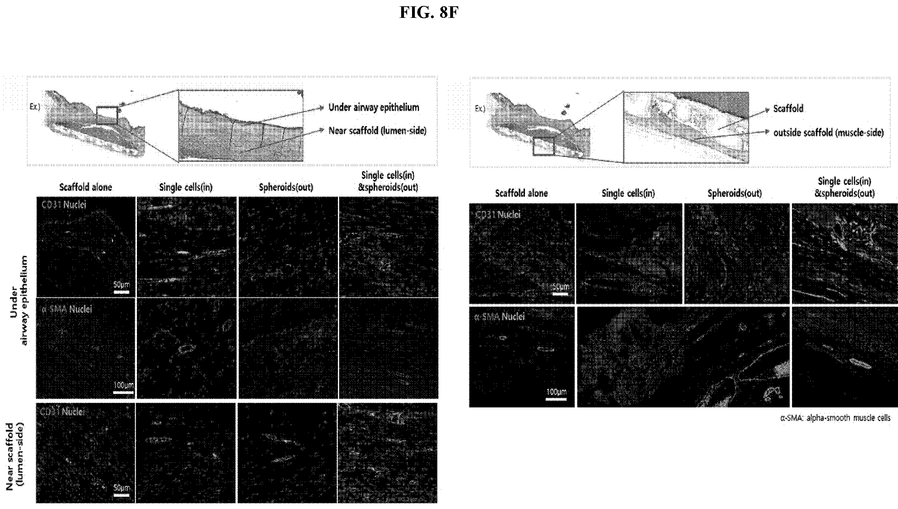

The biggest obstacles in conventional attempts at tracheal reconstruction were the absence of new blood vessels, and a low survival rate due to the resulting stenosis. To overcome this, the present invention introduced stem cell spheroids with an enhanced angiogenesis-inducing factor expression function, and confirmed their efficacy in Experimental Example 1. To evaluate new blood vessels formed in regenerated tracheal tissue, CD31 and $\alpha$-SMA immunostaining was conducted. As a result of confirming the distribution of CD31-positive cells at the bottom of the regenerated airway mucosa layer in the scaffold, as shown on the left side of FIG. 8F, in two groups where 2D-cultured stem cells were applied inside the scaffold, many distinct tubular blood vessels were observed, and compared to the case where a scaffold alone group in which tubular CD31-positive cells were not confirmed, new tubular blood vessels were observed also in the experimental group where only stem cell spheroids were applied outside the scaffold. In addition, through $\alpha$-SMA staining performed to confirm developed vascular distribution, the vascular distribution was clearly observed in the two groups where the 2D-cultured stem cells were applied inside the scaffold, it was found that applying stem cells inside the scaffold is important for inducing neovascularization during the regeneration of the airway mucosa. Moreover, since the stem cell spheroids with an enhanced angiogenesis-inducing factor expression function were applied outside the scaffold, blood vessels formed in the tissue outside the scaffold were also observed, and therefore it was confirmed that the largest amount of CD31-positive cells were observed in the two experimental groups where the stem cell spheroids were applied outside the scaffold, and $\alpha$-SMA-positive cells, indicating developed blood vessels, were also observed and are formed in a distinct tubule shape, as shown on the right side of FIG. 8F. The above results confirmed the angiogenesis-inducing efficacy of the stem cell spheroids.

### Experimental Example 4. Confirmation of reconstructive effect of aging vocal folds

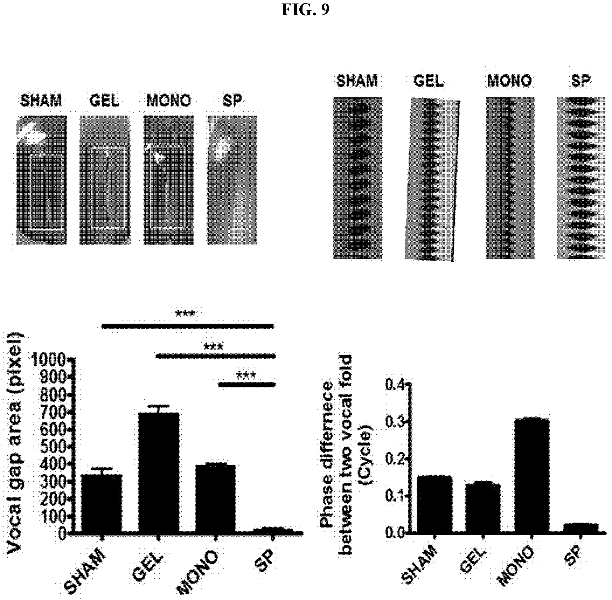

To confirm the reconstructive effect of cell spheroids on aging vocal folds, on day 3 of culture, 35 AD-MSC spheroids with a diameter of 200 $\mu$m were evenly mixed with hyaluronic acid hydrogel (HA gel, 1 wt%), and then injected into the vocal folds of an 18-month-old aging rat (Sprague Dawley rat, male) using a 25G puncture needle. As a control, a case where only HA gel was injected (GEL) and a case where the same number of single cells were injected (MONO) were prepared. In addition, one month later, the degree of functional reconstruction of the vocal folds was determined. In more detail, to determine the degree of functional reconstruction of the vocal folds, after photographing the movement of the vocal folds using a high-speed camera, kymographic images were obtained using the Meta-Morph analysis software, and then the gap area and the degree of vocal fold closure (gap difference), observed when the vocal folds were closed as tight as possible, were evaluated. Hereinafter, all animal experiments were conducted with the approval of the Institutional Animal Care and Use Committee. Afterward, all experiments were repeated at least three times, and the results are expressed as mean $\pm$ standard deviation. Statistical significance between two groups was confirmed by the Student's t-test, and statistical significance between three or more groups was confirmed using one-way ANOVA following Bonferroni's comparison test. * indicates P < 0.05, ** indicates P < 0.01, and *** indicates P < 0.001. The results are shown in FIG. 9.

As shown in FIG. 9, in the experimental group (SP) injected with stem cell spheroids, it was confirmed that the vocal folds were completely closed, whereas in the group injected with HA gel or single cells, a gap was wide and the vocal folds were not completely closed. In addition, the result of examining the degree of closure between the left and right vocal folds also showed that only when the stem cell spheroids were injected, the vocal folds were well closed. The above results show that the stem cell spheroids promote tissue regeneration and thus the function of aging vocal folds was reconstructed.

### Experimental Example 4. Verification of tissue damage delaying efficacy using animal models with ischemic limb disease

Animal models with ischemic limb disease were fabricated as reported in a previous study (Nat Protoc. 2009;4(12):1737-46.). Briefly, after a surgical skin incision (5 mm), the femoral artery and vein were bluntly dissected and separated from the inguinal skin fold just proximal to the vascular outline visible through the mouse skin. Then, a surgical suture was inserted under the femoral artery, and the distal femoral artery from the deep branch was ligated using a triple surgical knot, and the skin was sutured.

### 4-1. Confirmation of vascular regeneration

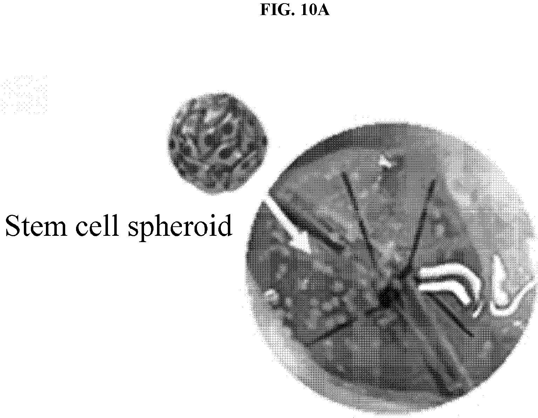

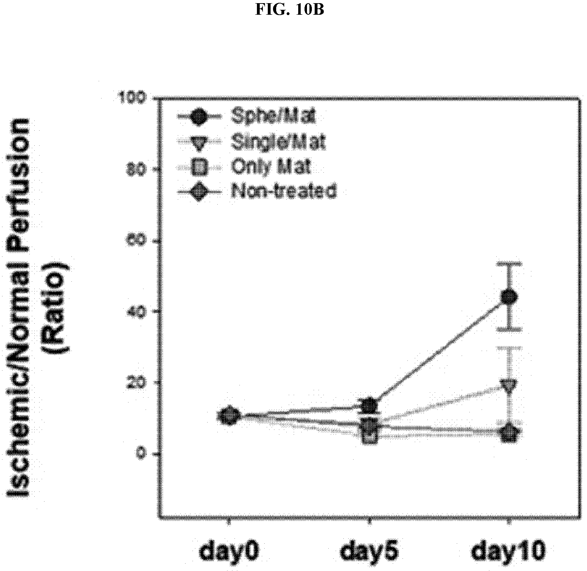

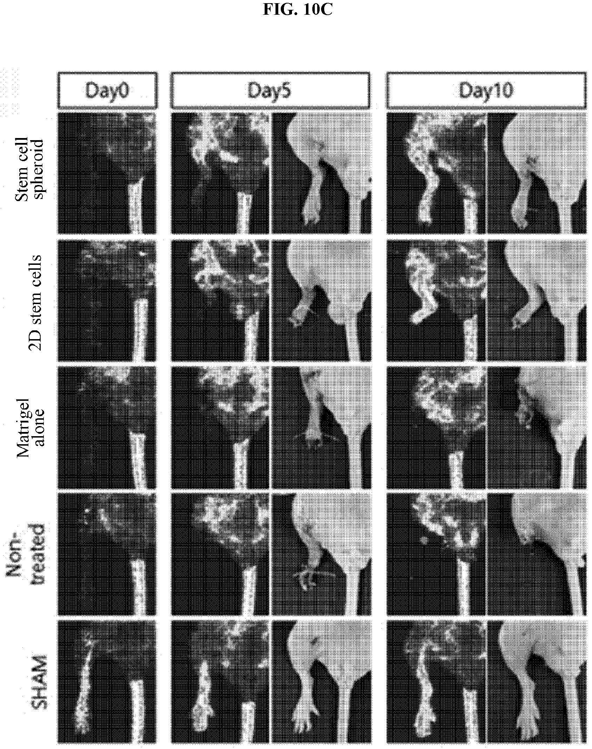

As shown in FIG. 10A, the damaged vascular area was covered with growth factor-reduced Matrigel in which optimized embryonic stem cell-derived mesenchymal stem cell spheroids were embedded, and then it was verified whether angiogenic factors expressed by the stem cell spheroids exhibited vascular damage-delaying efficacy. As a result, as shown in FIGS. 10B and 10C, it was confirmed that, on day 5 after transplantation, there was no significant difference in blood flow measurement between experimental groups, but the feet of the animals in all experimental groups excluding the experimental group transplanted with stem cell spheroids were damaged. On day 10 after transplantation, it was confirmed that blood flow was restored in the animal legs transplanted with stem cell spheroids and 2D-cultured stem cells. However, when the stem cell spheroids were transplanted, the foot shape was maintained and the blood flow was restored, whereas when the 2D-cultured stem cells were transplanted, the shape of the initially damaged foot was not restored. From this, it was confirmed that not only was the progression of ischemic lower limb disease delayed through stem cell spheroid transplantation, but damaged blood vessels were also regenerated.

**4-2. Confirmation of muscular damage delay and muscle regeneration**

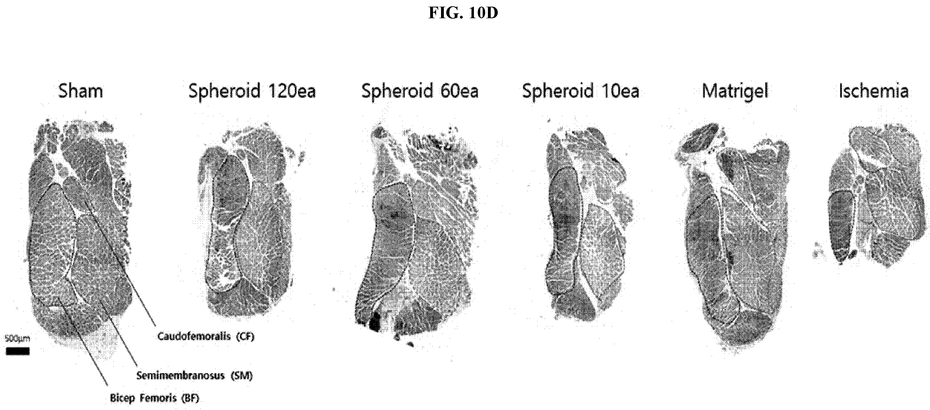

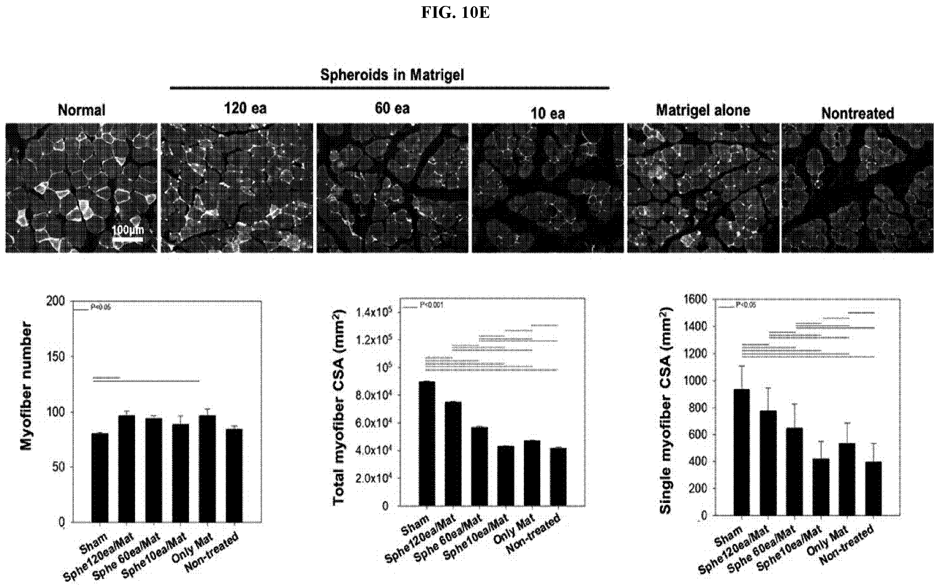

To verify muscular damage-delaying and muscular regenerating efficacies, histological evaluation was performed. As a result of H&E staining (FIG. 10D) and immunostaining (FIG. 10E), compared to an untreated experimental group, in the experimental group transplanted with stem cell spheroids, the total muscle cross-sectional area was wider. In addition, it was confirmed that the cross-sectional area (CSA) of one muscle fascicle was also wide. As the number of transplanted stem cell spheroids increased, the cross-sectional area of the muscle increased, indicating that the transplanted stem cell spheroids promoted muscle regeneration.

**4-3. Confirmation of induction of angiogenesis**

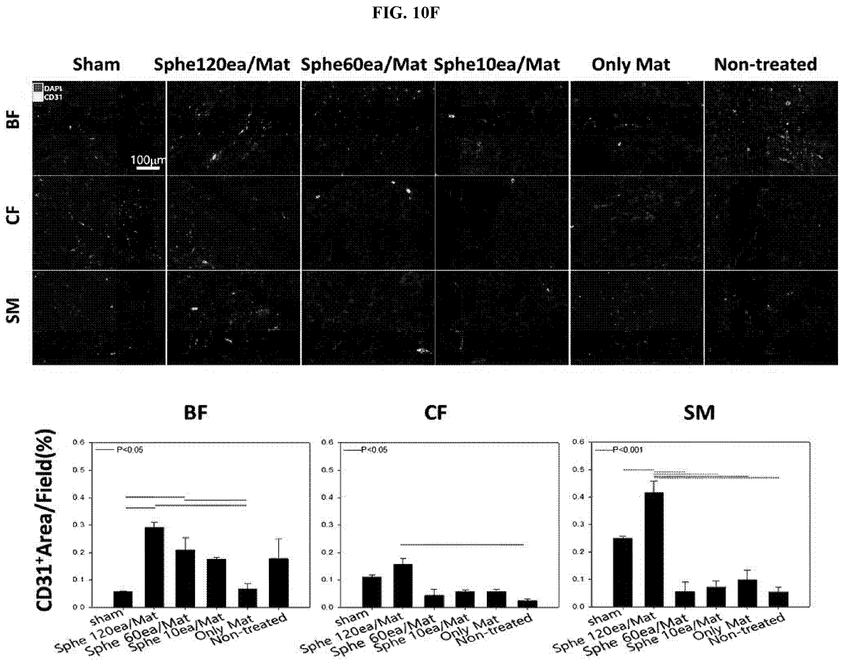

To evaluate the efficacy of transplanted stem cell spheroids in inducing angiogenesis, immunostaining with CD31 was used to detect vascular endothelial cells. As a result, as shown in FIG. 10F, compared to the untreated experimental group, in the experimental group transplanted with stem cell spheroids, the CD31-positive cell area was wider, indicating a large distribution of new blood vessels. In addition, considering that the distribution was larger than that of normal tissue, it was found that angiogenesis was promoted for tissue regeneration, and that the angiogenesis was induced by angiogenesis-promoting factors secreted by stem cell spheroids. Moreover, it can be seen that, as angiogenesis was promoted, the muscle regeneration-enhancing effect was induced in the experimental group transplanted with stem cell spheroids confirmed in Experimental Example 4-2.

**Experimental Example 5. Verification of skin defect regeneration efficacy**

Balb/c-nude mice (male, 13 months) were used to verify the skin defect regeneration efficacy of stem cell spheroids. As aging animals were used, their own tissue regeneration function was degraded due to aging, so it was expected that a tissue regeneration speed would be enhanced by bioactive factors expressed by stem cell spheroids.

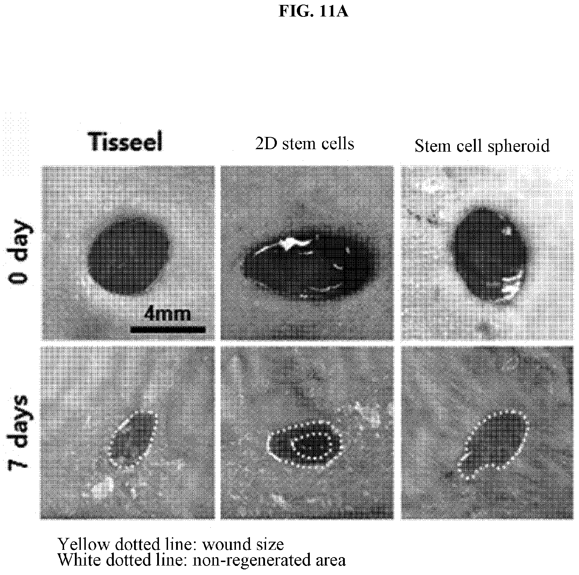

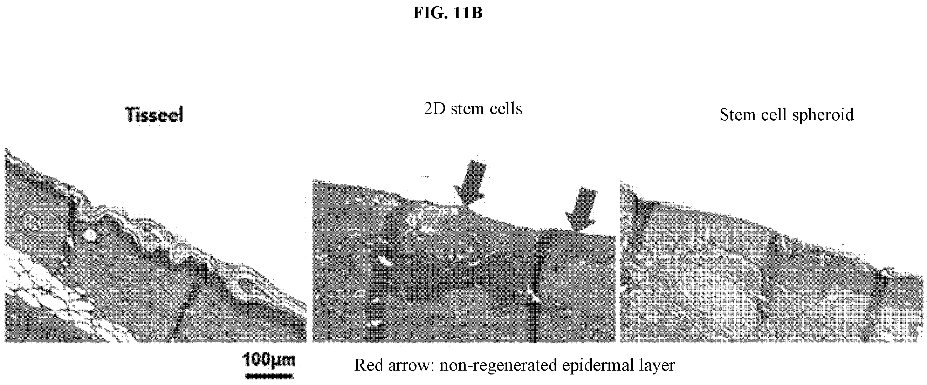

After creating a skin defect with a diameter of 4 mm on the dorsal skin of an aging mouse using a biopsy punch, defect areas were treated through Tisseel (fibrin sealant, Baxter Healthcare Corp., Deerfield, IL) treatment, Tisseel treatment after the application of 2D-cultured stem cells, and Tisseel treatment after the application of embryonic stem cell-derived mesenchymal stem cell spheroids. Here, Tisseel was used to repair the defect areas and to fix the stem cell spheroids applied to the defect areas thereto. After 7 days of treatment, as a result of gross examination, shrinkage of the wound area was detected when only Tisseel was treated as shown in FIG. 11A. When only 2D-cultured stem cells were treated, it was confirmed that the tissue was not completely regenerated even after 7 days of treatment. However, when stem cell spheroids were treated, the degree of wound shrinkage was significantly lower than that of the only Tisseel treated group, and the wound was completely healed. In addition, as a result of H&E staining of the regenerated tissue recovered 7 days after treatment, as shown in FIG. 11B, when stem cell spheroids were treated, the epidermis layer was completely covered and thickly formed, confirming that not only was the speed of tissue regeneration improved, but the regenerated tissue was also regenerated most similarly to the original tissue.

**Experimental Example 6. Verification of fat reconstructive efficacy**

The fat pad of a Balb/c-nude mouse (male, 13 months) was incised, and each of Matrigel, 2D-cultured stem cell-embedded Matrigel, and stem cell spheroid-embedded Matrigel was transplanted. The fat reconstructive efficacy of the stem cell spheroids was then verified. Here, Matrigel with reduced growth factors was used to induce fat regeneration by bioactive factors expressed by stem cell spheroids. In addition, the fat reconstruction/regeneration rate of stem cell spheroids was confirmed by using male experimental animals with slow fat reconstruction and regeneration rates.

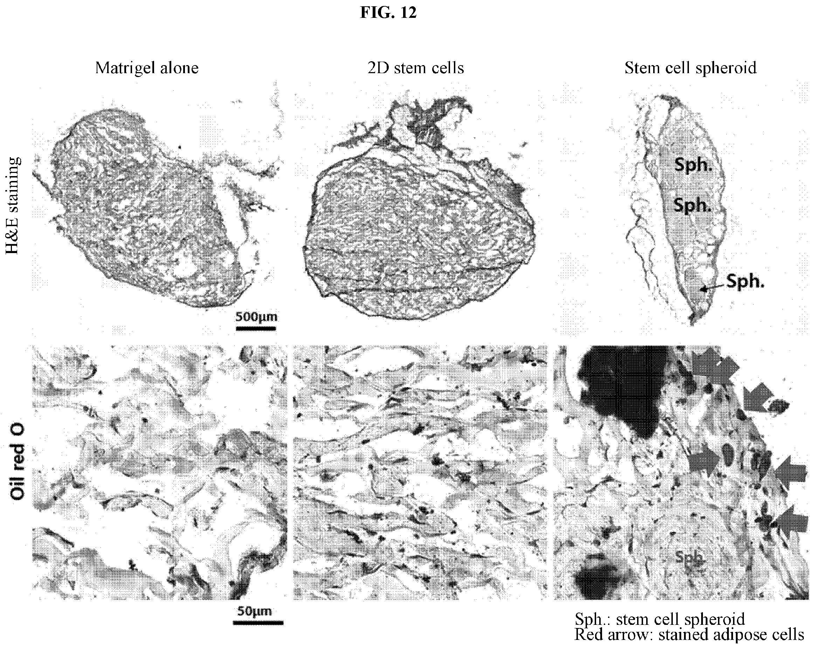

After 14 days of transplantation of each experimental group into the fat pad of the mouse, the transplanted samples were recovered to perform histological evaluation. As shown in FIG. 12, as a result of confirming integration through H&E staining, in the 2D-cultured stem cell-embedded Matrigel experimental group, it was difficult to confirm transplanted stem cells, whereas in the embryonic stem cell-derived mesenchymal stem cell spheroid-embedded Matrigel experimental group, the morphology of the transplanted stem cell spheroids was confirmed. In addition, to confirm whether adipose cells migrated from the surrounding tissue to each experimental group transplanted for fat reconstruction/regeneration by staining the oil drop of the adipose cells through Oil red O staining, as shown in FIG. 12, when only Matrigel and 2D-cultured stem cell-embedded Matrigel were transplanted, oil drops were not identified, whereas when stem cell spheroid-embedded Matrigel was transplanted, red-stained oil drops were identified around the stem cell spheroids. Therefore, it was confirmed that bioactive factors expressed by stem cell spheroids induce the migration of surrounding adipose cells, thereby exhibiting fat reconstruction/regeneration-inducing efficacy.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative in all aspects and not restrictive.

[Industrial Application]

The stem cell spheroid according to the present invention exhibits excellent effects on the regeneration of various tissues such as salivary gland, trachea, vocal cord, muscle, skin, and fat tissue through actions such as angiogenesis, and is expected to be usefully utilized as a cell therapy agent for various tissue disorders, thus having industrial applicability.

**Claims**

1. A composition for promoting tissue regeneration, comprising stem cell spheroids as an active ingredient.

2. The composition of claim 1, wherein the stem cells are one or more selected from the group consisting of bone marrow-derived mesenchymal stem cells, adipose-derived mesenchymal stem cells, embryonic stem cell-derived mesenchymal stem cells, tonsil-derived mesenchymal stem cells, and induced pluripotent stem cell-derived mesenchymal stem cells.

3. The composition of claim 1, wherein the tissue is one or more selected from the group consisting of skin, fat, salivary glands, the trachea, airways, the vocal folds, and muscles.

4. The composition of claim 1, the composition further comprises a scaffold for tissue engineering.

5. The composition of claim 1, wherein the stem cell spheroids exhibit enhanced expression of bioactive factors associated with promotion of tissue regeneration.

6. The composition of claim 5, wherein the bioactive factors are one or more selected from the group consisting of vascular endothelial growth factors (VEGFs), matrix metalloproteinases (MMPs), fibroblast growth factors (FGFs), epidermal growth factors (EGFs), hepatocyte growth factor (HGF), platelet-derived growth factor (PDGF), placental growth factor (PIGF), granulocyte-macrophage colony-stimulating factor (GM-CSF), transforming growth factor beta (TGF-β), and angiopoietin.

7. The composition of claim 1, wherein the stem cells promote angiogenesis.

8. The composition of claim 1, wherein the stem cell spheroids have diameters ranging from 100 to 500 μm.

9. A cell therapeutic agent for promoting tissue regeneration, comprising stem cell spheroids as an active ingredient.

10. A method for promoting tissue regeneration, comprising:
administering a composition comprising stem cell spheroids as an active ingredient to a subject in need thereof.

11. A use of a composition comprising stem cell spheroids as an active ingredient for the promotion of tissue regeneration.

12. A use of a composition comprising stem cell spheroids as an active ingredient for the preparation of a formulation for promoting tissue regeneration.

EP 4 620 494 A1

**FIG. 1**

FIG. 2

**150 μm spheroid**

Red: BMdMSC
White: iPSdMSC
Green: AdMSC

$y = -0.3694x + 6.6138$
$R^2 = 0.7857$

$y0 = 0.29$ to $5.14$

**200 μm spheroid**

$y = -0.6368x + 13.0818$
$R^2 = 0.9751$

$y0 = 1.71$ to $8.57$

**250 μm spheroid**

$y = -1.5734x + 30.2454$
$R^2 = 0.9747$

$y0 = 4.29$ to $17.14$

EP 4 620 494 A1

**FIG. 3**

**FIG. 4**

| Mold | Agar mold, 81ea | | | PDMS mold, 169ea |
|---|---|---|---|---|
| Target diameter (μm) | 150 | 200 | 250 | 250 |
| Average diameter (μm) | 156.5 ± 19.4 | 201.0 ± 25.5 | 242.6 ± 29.9 | 239.8 ± 11.7 |
| Image of spheroids at day 3 of spheroidal formation | | | | |

EP 4 620 494 A1

FIG. 5A

**FIG. 5B**

**FIG. 5C**

**FIG. 5D**

FIG. 6A

Co-culture with stem cell spheroid

| Without antiVEGF | 0.53 pM of antiVEGF | 1.07 pM of antiVEGF | HUVECs alone |

FIG. 6B

**FIG. 7A**

Normal   Tumor   Tumor/Sphe.   Tumor/IR   Tumor/IR/Sphe.

scale bar: 20μm

**FIG. 7B**

scale bar: 20μm

**FIG. 7C**

Normal      Tumor      Tumor/Sphe.      Tumor/IR      Tumor/IR/Sphe.

scale bar: 20μm

FIG. 8A

EP 4 620 494 A1

FIG. 8B

1. Tracheal partial defect animal model

2. Implantation of tracheal scaffold

Yellow asterisk: scaffold/cell therapy agent complex

After 14 weeks of implantation

Scaffold alone

2D stem cells (in)

Spheroid (out)

2D stem cells (in) & spheroid (out)

FIG. 8C

Yellow arrow: point at which airway mucosa is determined to have regenerated by 50% or more.

FIG. 8D

CK: cytokeratin, PCNA: proliferating cell nucleic acid

EP 4 620 494 A1

FIG. 8E

FIG. 8F

**FIG. 9**

**FIG. 10A**

Stem cell spheroid

EP 4 620 494 A1

**FIG. 10B**

**FIG. 10C**

FIG. 10D

Sham  Spheroid 120ea  Spheroid 60ea  Spheroid 10ea  Matrigel  Ischemia

500μm

Caudofemoralis (CF)

Semimembranosus (SM)

Bicep Femoris (BF)

EP 4 620 494 A1

**FIG. 10E**

**FIG. 10F**

**FIG. 11A**

Yellow dotted line: wound size
White dotted line: non-regenerated area

**FIG. 11B**

Tisseel                    2D stem cells                    Stem cell spheroid

100μm          Red arrow: non-regenerated epidermal layer

EP 4 620 494 A1

**FIG. 12**

Sph.: stem cell spheroid
Red arrow: stained adipose cells

## INTERNATIONAL SEARCH REPORT

| International application No. |
|---|
| **PCT/KR2023/018404** |

**A. CLASSIFICATION OF SUBJECT MATTER**

**A61L 27/38**(2006.01)i; **A61L 27/36**(2006.01)i

According to International Patent Classification (IPC) or to both national classification and IPC

**B. FIELDS SEARCHED**

Minimum documentation searched (classification system followed by classification symbols)

A61L 27/38(2006.01); A61K 35/12(2006.01); A61K 35/28(2006.01); A61K 35/32(2006.01); C12N 13/00(2006.01); C12N 5/071(2010.01); C12N 5/077(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 줄기세포(stem cell), 스페로이드(spheroid), 조직재생(neogenesis), 세포치료제 (Cell therapy), 혈관신생(앤지오제네시스, angiogenesis)

**C. DOCUMENTS CONSIDERED TO BE RELEVANT**

| Category* | Citation of document, with indication, where appropriate, of the relevant passages | Relevant to claim No. |
|---|---|---|
| X | KR 10-2016-0000030 A (DANKOOK UNIVERSITY CHEONAN CAMPUS INDUSTRY ACADEMIC COOPERATION FOUNDATION) 04 January 2016 (2016-01-04)<br>See claims 1-3 and paragraphs [0009], [0013]-[0038] and [0061]-[0064]. | 1-9,11,12 |
| A | KR 10-2021-0021165 A (AJOU UNIVERSITY INDUSTRY-ACADEMIC COOPERATION FOUNDATION) 25 February 2021 (2021-02-25)<br>See claims 1-7. | 1-9,11,12 |
| A | KR 10-2022-0062167 A (THE CATHOLIC UNIVERSITY OF KOREA INDUSTRY-ACADEMIC COOPERATION FOUNDATION) 16 May 2022 (2022-05-16)<br>See claims 1-15. | 1-9,11,12 |
| A | KR 10-2021-0067988 A (SUNGKWANG MEDICAL FOUNDATION et al.) 08 June 2021 (2021-06-08)<br>See claims 1-8. | 1-9,11,12 |

☑ Further documents are listed in the continuation of Box C.   ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **28 February 2024** | **28 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/018404** |

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2021-0059809 A (INCHEON NATIONAL UNIVERSITY RESEARCH & BUSINESS FOUNDATION et al.) 26 May 2021 (2021-05-26)<br>See claims 1-20. | 1-9,11,12 |
| A | KR 10-1922346 B1 (SEOUL NATIONAL UNIVERSITY HOSPITAL et al.) 26 November 2018 (2018-11-26)<br>See claims 1-9. | 1-9,11,12 |

**INTERNATIONAL SEARCH REPORT**

| International application No. |
| --- |
| **PCT/KR2023/018404** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **10**
   because they relate to subject matter not required to be searched by this Authority, namely:

   Claim 10 pertains to a method for treatment of the human body by means of a composition (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2023/018404**

| Patent document cited in search report | | | Publication date (day/month/year) | Patent family member(s) | | | Publication date (day/month/year) |
|---|---|---|---|---|---|---|---|
| KR | 10-2016-0000030 | A | 04 January 2016 | KR | 10-1719870 | B1 | 27 March 2017 |
| | | | | KR | 10-1816964 | B1 | 11 January 2018 |
| | | | | KR | 10-2016-0000029 | A | 04 January 2016 |
| | | | | WO | 2015-199416 | A1 | 30 December 2015 |
| KR | 10-2021-0021165 | A | 25 February 2021 | | None | | |
| KR | 10-2022-0062167 | A | 16 May 2022 | KR | 10-2533124 | B1 | 16 May 2023 |
| KR | 10-2021-0067988 | A | 08 June 2021 | KR | 10-2021-0019306 | A | 22 February 2021 |
| | | | | KR | 10-2384953 | B1 | 11 April 2022 |
| | | | | US | 2022-296649 | A1 | 22 September 2022 |
| | | | | WO | 2021-029651 | A1 | 18 February 2021 |
| KR | 10-2021-0059809 | A | 26 May 2021 | | None | | |
| KR | 10-1922346 | B1 | 26 November 2018 | | None | | |

Form PCT/ISA/210 (patent family annex) (July 2022)

**REFERENCES CITED IN THE DESCRIPTION**

**Patent documents cited in the description**

- KR 1020220153034

- KR 1020230158492

**Non-patent literature cited in the description**

- *Nat Protoc.*, 2009, vol. 4 (12), 1737-46